Europäisches Patentamt

European Patent Office

Office européen des brevets

(19)

(11) Publication number: **0 375 785 B1**

## (12) EUROPEAN PATENT SPECIFICATION

(45) Date of publication of patent specification: **06.07.94**

(21) Application number: **89905208.8**

(22) Date of filing: **24.04.89**

(86) International application number:
**PCT/JP89/00430**

(87) International publication number:
**WO 89/10186 (02.11.89 89/26)**

(51) Int. Cl.⁵: **B01F 17/00**, B01F 17/14,
B01F 17/38, A23J 7/00,
A61K 7/00, A61K 31/685,
A61K 47/00

The file contains technical information submitted
after the application was filed and not included in
this specification

(54) LIPID COMPOSITION HAVING ENOUGH SAFETY AND STRONG SURFACE ACTIVITY.

(30) Priority: **28.04.88 JP 106171/88**
**04.06.88 JP 138150/88**
**04.06.88 JP 138151/88**

(43) Date of publication of application:
**04.07.90 Bulletin 90/27**

(45) Publication of the grant of the patent:
**06.07.94 Bulletin 94/27**

(84) Designated Contracting States:
**AT BE CH DE FR GB IT LI LU NL SE**

(56) References cited:
**WO-A-86/05694**
**JP-A- 6 245 336**
**JP-A- 6 354 926**
**JP-A-58 133 826**

(73) Proprietor: **ASAHI DENKA KOGYO KABUSHIKI KAISHA**
**2-35, Higashiogu 7-chome**
**Arakawa-ku Tokyo 116(JP)**

(72) Inventor: **FUJITA, Satoshi Asahi Denka Kogyo Kabushiki Kaisha**
**2-35, Higashiogu 7-chome**
**Arakawa-ku Tokyo 116(JP)**

(74) Representative: **Patentanwälte Grünecker, Kinkeldey, Stockmair & Partner**
**Maximilianstrasse 58**
**D-80538 München (DE)**

EP 0 375 785 B1

**Description**

BACKGROUND OF THE INVENTION

Field of the Invention:

This invention relates to a lipid composition having a high safety and intense surface activities including permeability, wettability and spreadability.

Description of the Prior Art:

Examples of surfactants which are highly safe and widely used in the fields, such as, foods, feeds, cosmetics, sanitary goods, drugs and agricultural chemicals include glycerophospholipids (lecithin) which are optionally fractionated, hydrogenated or enzymatically treated, natural or synthetic glycerol fatty acid monoesters, tartaric acid, malic acid, acetic acid, lactic acid and succinic acid derivatives of glycerol fatty acid monoesters, sorbitan fatty acid esters, sucrose fatty acid esters, propylene glycol fatty acid esters, polyglycerol fatty acid esters, condensed ricinoleate of polyglycerol fatty acid esters, stearoyl-2-lactylate, polyoxyethylene sorbitan fatty acid esters, polyoxyethylene fatty acid esters, sodium dioctyl sulfosuccinate, sodium dodecyl sulfate and mono- and diacylphosphatidic acids. Since these surfactants are relatively safe, they have been used in many countries. However acceptable intakes per day are determined for those other than glycerophospholipids (lecithin) and mono- and diglycerides of fatty acids. Further, the utilization of many of these surfactants is approved only for limited purposes, and unlimited application of these surfactants has never been approved in any country at present.

Although monoacylglycerophospholipids obtained by treating soybean phospholipids with phospholipase A-2, in particular, lyso phosphatidylcholine and highly pure sucrose monofatty acid esters are highly safe and have excellent surface activities, the properties of these surfactants, such as permeability wettability and spreadability, are yet unsatisfactory.

Furthermore, these surfactants are also unsatisfactory from the viewpoint of the acceleration of the transintestinal, percutaneous and transmucosal absorption of drugs.

Various surface active compositions have been disclosed in, for example, Japanese Patent Publication No. 51241/1984, U.S. Patent No. 3,388,999, Danish Patent No. 101,649, and U.S. Patent No. 3,661,795 and No. 3,549,382. However none of these compositions has satisfactory properties.

Published Japanese Translation of PCT Patent Application from other Countries No. 502891/1987 has disclosed a composition for accelerating the absorption of an orally administered drug in vivo which comprises (a) at least one fatty acid having 14 to 18 carbon atoms; (b) at least one monoglyceride comprising glycerol and a fatty acid having 14 to 18 carbon atoms; and (c) lysophosphatidylcholine containing a fatty acid component having 14 to 18 carbon atoms; optionally together with (d) a drug. In this PCT Patent Application, palmitoleic, oleic, linoleic and linolenic acids and saturated fatty acids having 14 to 18 carbon atoms are cited as the examples of the fatty acids consituting the abovementioned components (a), (b) and (c).

However the composition described in the above PCT Patent Application is one which aims at distributing an orally administered substance. Namely, it is not the main object of said composition to achieve surface activities. Therefore the composition disclosed above cannot give sufficiently intense surface activities. For example, a composition comprising a saturated fatty acid having 14 to 18 carbon atoms, as defined in the component (a) of the composition disclosed in the above PCT Patent Application, has extremely poor surface activities such as permeability, wettability and spreadability.

SUMMARY OF THE INVENTION

It is an object of the present invention to provide a lipid composition having a high safety and intense surface activities such as permeability, wettability, adhesivity and spreadability (immersional and adhesional wettability).

As the result of extensive studies, the present inventors found that a composition comprising glycerophospholipid(s) partially hydrolyzed by phospholipase A-2, monoglyceride(s) of unsaturated fatty acid(s) and/or monoglyceride(s) of medium-chain fatty acid(s) has a high safety and intense surface activities; and that said composition can be economically obtained. Thus, they have applied for a patent on said composition (cf. Japanese Patent Application No. 207093/1987 and No. 240205/1987). However said composition is not always satisfactory in the permeability and spreadability.

2

As the result of subsequent studies, the present inventors have found that the object of the present invention can be achieved by using the following lipid composition, thus completing the present invention.

Then, the present invention provides a lipid composition having a high safety and significant surface activities which essentially comprises:

(A) acylglycerophospholipid(s),
(B) fatty acid monoglyceride(s), and
(C) fatty acid(s),
together with:
(D) bile acid(s) and/or alkali salt(s) thereof, and satisfies either one of the following requirements 1 to 3.

The WO-A-8605694 describes compositions of components (A), (B) and (C).

Requirement 1:

The component (A) comprises acylglycerophospholipid(s) (A-1) containing at least 10% by weight of monoacylglycerophospholipid(s).

The component (B) comprises fatty acid monoglyceride(s) (B-1) comprising monoglyceride(s) of unsaturated fatty acid(s) having 14 to 22 carbon atoms or mixed fatty acid monoglycerides comprising said unsaturated fatty acid(s) and saturated fatty acid(s) having 14 to 22 carbon atoms, having an iodine value of 30 or above and containing at least 40% by weight of monoester(s).

The component (C) comprises at least one fatty acid (C-1) selected from the group consisting of fatty acids (C-1-1) comprising unsaturated fatty acid(s) having 16 to 22 carbon atoms and a mixture of said unsaturated fatty acids with saturated fatty acid(s) having 14 to 18 carbon atoms and having an iodine value of 50 or above, and medium-chain fatty acids (C-1-2) having 10 to 13 carbon atoms.

The ratios by weight among these components are represented by the following equations:

(B-1)/(A-1) = 10/90 to 90/10,
(C-1)/(B-1) = 20/100 to 250/100, and
(C-1)/((A-1) + (B-1)) = 10/100 to 200/100.

When ((B-1) + (C-1))/(A-1) exceeds 4, the composition comprises the component (D) in an amount of 5 to 100% by weight based on the total amount of ((A-1) + (B-1) + (C-1)).

Rrequirement 2:

The component (A) comprises lysophosphatidylcholine (A-2).

The component (B) comprises monoglyceride(s) (B-2) of unsaturated fatty acid(s) having 16 to 22 carbon atoms.

The component (C) comprises fatty acid(s) (C-2) having 9 to 13 carbon atoms.

The ratios by weight among these components are represented by the following equations:

(B-2)/(A-2) = 10/90 to 90/10,
(C-2)/(B-2) = 20/100 to 200/100, and
(C-2)/((A-2) + (B-2)) = 10/100 to 200/100.

When ((B-2) + (C-2))/(A-2) exceeds 5, the composition comprises the component (D) in an amount of 5 to 100% by weight based on the total amount of ((A-2) + (B-2) + (C-2)).

Requirement 3:

The component (A) comprises acylglycerophospholipid(s) (A-3) containing at least 10% by weight of monoacylglycerophospholipid(s).

The component (B) comprises monoglyceride(s) (B-3) of fatty acid(s) having 8 to 13 carbon atoms.

The component (C) comprises saturated fatty acid(s) having 8 to 18 carbon atoms and/or unsaturated fatty acid(s) having 14 to 22 carbon atoms (C-3).

The ratios by weight among these components are represented by the following equations.

(B-3)/(A-3) = 10/90 to 95/5,
(C-3)/(B-3) = 20/100 to 250/100, and

EP 0 375 785 B1

(C-3)/((A-3) + (B-3)) = 10/100 to 200/100.

When ((B-3) + (C-3))/(A-3) exceeds 6, the composition should further comprise the component (D) in an amount of 5 to 100% by weight based on the total amount of ((A-3) + (B-3) + (C-3)).

The composition of the present invention is a lipid composition which has a remarkably high safety and intense surface activities such as permeability, wettability and spreadability.

Namely, a aqueous solution of the composition of the present invention has such excellent surface activities that

(1) unless directly administered into blood or cells, it exerts mild and safe effects on living organisms both in endothelial and integumentary systems, since it exclusively consists of natural lipids.

(2) it is superior in permeability and wettability,

(3) it is highly spreadable on the surfaces of animals or plants,

(4) it promotes the dispersion of solid microparticles in water, and

(5) it is effective when is used in the emulsification of oily substances.

DETAILED DESCRIPTION OF THE INVENTION

First, a lipid composition satisfying the requirement 1 will be described. The acylglycerophospholipids (A-1), namely the component (A), are obtained by partially deacylating glycerophospholipids originating from natural substances. Any mixture may be employed therefor without restriction, so long as it contains at least 10% by weight of monoacylglycerophospholipid(s) and substantially comprises mono- and diacylglycerophospholipids. From the practical viewpoint, it may be obtained by partially hydrolyzing by-products formed in the production of fat or oil from lipid cereals, for example, soybean phospholipids or rapeseed phospholipids or diacylglycerophospholipids obtained from, for example, yolk, peanuts or corn germs by using an acid such as sulfuric or phosphoric acid. Still preferably, partially decomposed acylglycerophospholipids obtained by hydrolyzing the abovementioned materials with phospholipase A-2 originating from pancreation or snake venom and removing the coexisting impurities including fatty acids and triglycerides through a treatment with acetone etc. may be employed therefor.

The abovementioned acylglycerophospholipid(s) (A-1) may preferably comprise a mixture of various phosphatides having mono or diacyl groups. Namely, a mixture comprising monoacyl and diacyl phosphatidylcholine, phosphatidylethanolamine, phosphatidylinositol, phosphatidic acid and phosphatidylserine is preferably employed therefor.

Furthermore, it is preferable that the above acylglycerophospholipids (A-1) comprise 75% by weight or less of compounds having the same group, e.g., choline or ethanolamine, to be bound to a phosphate group. When these lipids are fractionated by using a solvent such as an aqueous alcohol or by a conventional method to thereby elevate the content of compounds having the same group to be bound to a phosphate group to 75% or above, the desired effects cannot be satisfactorily achieved in some cases. Examples of such an undesirable mixture include those comprising mainly monoacylphosphatidylcholine or monoacylphosphatidylcholine and diacylphosphatidylcholine.

It is preferable that the content of monoacyl compound(s) based on the total amount of the monoacyl and diacyl compounds in the abovementioned acylglycerophospholipids (A-1) is at least 10% by weight, still preferably at least 25% by weight. When said content is lower than 10% by weight, the effects of the present invention can be hardly achieved. Although the upper limit of the content of the monoacyl compounds is not specifically defined, the use of 80% by weight or more of the same cannot remarkably elevate the effects and a mixture rich in mono acryl compounds is expensive. From these points of view, it is economically disadvantageous to use 80% by weight or more of these compounds.

Examples of the phospholipids useful as the starting material in the preparation of the abovementioned acylglycerophospholipidS (A-1) include soybean, rapeseed and yolk phospholipids. However other phospholipids are also available therefor and those lacking some components other than phosphatidylcholine may also be used.

The abovementioned acylglycerophospholipids (A-1) may be analyzed by a number of methods. For example, the component A-1 may be analyzed by subjecting the same to thin layer chromatography and examining each spot thus obtained; by using a TLC-FID analyzer (Iatro Scan method); by high-performance liquid chromatography; and by combining these procedures.

The fatty acid monoglyceride(s) (B-1), namely the component (B), may be either natural ones or ones obtained through the hydrolysis of oil and fat with lipase or glycerolysis thereof. Alternately, synthetic ones mad be used therefor. Fatty acid monoglycerides synthesized from glycerol and fatty acids, which are obtained by hydrolysis of oil and fat, and are obtained by distillation or a solvent-treatment, and by partial

4

hydrogenation thereof, may be commonly employed therefor.

The abovementioned fatty acid monoglycerides (B-1) may be either 1-, 3-glycerides or 2-glycerides with respect to the carbon atoms of the glycerol.

The abovementioned fatty acid monoglyceride(s) (B-1) usually comprises monoglyceride(s) of unsaturated fatty acid(s) having 14 to 22 carbon atoms or mixed fatty acid monoglycerides comprising said unsaturated fatty acid(s) and saturated fatty acid(s) having 14 to 22 carbon atoms. The component (B-1) contains at least 40% by weight of monoester(s) having an iodine value of 30 or above. When the component (B-1) departs from the abovementioned range, the effects of the present invention can be hardly achieved. In particular, an iodine value lower than 30 would bring about an undesirably low permeability.

It is preferable that the fatty acid monoglyceride(s) (B-1) contains at least 70% by weight of monoester(s), since sufficient effects can be obtained in such a case. Distilled monoglycerides are particularly suitable for the composition of the present invention.

The fatty acid(s) (C-1), namely the component (C), may be at least one fatty acid selected from the group consisting of fatty acids (C-1-1) comprising unsaturated fatty acids having 16 to 22 carbon atoms and a mixture of said unsaturated fatty acids with saturated fatty acids having 14 to 18 carbon atoms and having an iodine value of 50 or above and medium-chain fatty acids (C-1-2) having 10 to 13 carbon atoms.

Examples of the unsaturated fatty acids having 16 to 22 carbon atoms to be used in the above component (C-1-1) include palmitoleic, oleic, linolenic, arachidonic and eicosapentaenoic acids. These long-chain unsaturated fatty acids may be mixed with saturated fatty acid(s) having 14 to 18 carbon atoms. In this case, however., it is necessary that the resulting fatty aid mixture have an iodine value of 50 or above. An increase in the content of the saturated long-chain fatty acid(s) in the composition of the present invention is undesirable, since the solubility of said composition in water as a micelle is lowered thereby. As a result, the composition becomes hardly soluble in water even when bile acid salts are added thereto.

The permeability or the composition of the present invention would increase with an increase in the iodine value of said fatty acid(s) (C-1-1). A fatty acid in a composition of present invention which has an iodine value less than 50 cannot give satisfactory effects. Unsaturated fatty acids having 14 or less or 23 or more carbon atoms rarely occur in nature.

The abovementioned medium-chain fatty acids (C-1-2) having 10 to 13 carbon atoms are also available as said fatty acid(s) (C-1). On the other hand, fatty acids having eight or less or 14 or more carbon atoms cannot give sufficient surface activities such as permeability and spreadability. When the component (C) mainly comprises said medium-chain fatty acids (C-1-2), those containing a small amount of fatty acid(s) having 14 or more carbon atoms, for example, coconut oil fatty acids or palm kernel fatty acids may be employed.

The composition of the present invention comprises the abovementioned acylglycerophospholipid(s) (A-1), fatty acid monoglyceride(s) (B-1) and fatty acid(s) (C-1) at a ratio by weight satisfying the following relationships:

(B-1)/(A-1) = 10/90 to 90/10,
(C-1)/(B-1) = 20/100 to 250/100, and
(C-1)/((A-1) + (B-1)) = 10/100 to 200/100,

preferably

(B-1)/(A-1) = 30/70 to 80/20,
(C-1)/(B-1) = 30/100 to 170/100, and
(C-1)/((A-1) + (B-1)) = 25/100 to 160/100.

When the ratio by weight of said fatty acid monoglyceride(s) (B-1) to said acylglycerophospholipid(s) (A-1), i.e., (B-1)/(A-1) is less than 10/90, the effects of the present invention cannot be satisfactorily achieved. when said ratio exceeds 90/10, on the other hand, the solubility of the composition in water would be undesirably lowered. When the ratio of said fatty acid(s) (C-1) to said fatty acid monoglyceride(s) (B-1), i.e., (C-1)/(B-1) is less than 20/100, the effects of the present invention cannot be satisfactorily achieved. When said ratio exceeds 250/100, on the other hand, the effects of the invention can never be improved any more. In addition, the solubility of the composition in water would be lowered in this case. When the ratio of said fatty acid(s) (C-1) to the total amount of said acylglycerophospholipid(s) (A-1) and fatty acid monoglyceride(s) (B-1), i.e., (C-1)/((A-1) + (B-1)) is lower than 10/100, the effects of the present invention cannot be satisfactorily achieved. When said ratio exceeds 20/100, on the other hand, the effects can never be improved any more and the solubility of the composition in water would be undesirably lowered.

5

The composition of the present invention further contains bile acid(s) and/or alkali salt(s) thereof (D), which will be referred to as the bile acids etc. (D) hereinafter, in an amount smaller than the total amount of the abovementioned acylglycerophospholipid(s) (A-1), fatty acid monoglyceride(s) (B-1) and fatty acid(s) (C-1) on a weight basis.

The bile acids etc. (D) may be selected from among mammal bile acids. Preferred examples thereof include one or more bile acids selected from among cholic, deoxycholic, chenodeoxycholic and lithocholic acids. Particularly preferable examples thereof include conjugated bile acids obtained by reacting the abovementioned bile acids with taurine and/or glycine and alkali metal salts of these bile acids.

The addition of the abovementioned bile acids etc. (D) enables stable solubilization of said fatty acid monoglyceride(s) (B-1) and fatty acid(s) (C-1), even when the content of monoacylglycerophospholipid(s) in the acylglycerophospholipid(s) (A-1) is relatively small.

When the content of the monoacylglycerophospholipid(s) in the abovementioned acylglycerophospholipid(s) (A-1) in the composition of the present invention is low or the content of the monoglyceride(s) in the abovementioned fatty acid monoglyceride(s) (B-1) is low, it is preferable to add the abovementioned bile acids etc. (D)

When the ratio by weight of the total amount of said fatty acid monoglyceride(s) (B-1) and fatty acid(s) (C-1) to said acylglycerophospholipid(s) (A-1), i.e., ((B-1) + (C-1))/(A-1) exceeds a certain level, it is necessary to add a solubilizer such as the bile acids etc. (D) thereof. This level is approximately ((B-1) + (C-1))/(A-1) = 4, though it would somewhat vary depending on the composition.

The upper limit of the amount of the bile acids etc. (D) to be added is not particularly specified. However no improvement is observed any more than this amount exceeds the total amount (weight) of the abovementioned acylglycerophospholipid(s) (A-1), fatty acid monoglyceride(s) (B-1) and fatty acid(s) (C-1). Therefore this level may be deemed as the upper limit from the economic viewpoint. On the other hand, the lower limit of the amount of the bile acids etc. (D) is 5% by weight of the total amount of the abovementioned acylglycerophospholipid(s) (A-1), fatty acid monoglyceride(s) (B-1) and fatty acid(s) (C-1), at which the aimed effects are observed.

The addition of the bile acids etc. (D) causes scarcely any change in the surface activities of the composition as far as the surface tension lowering effect and contact angle are concerned. On the other hand, the wetting time determined by the canvas disc method would be shortened thereby. However this effect is slight, even when the bile acids etc. (D) are added in an amount exceeding 50% by weight of the total amount of the abovementioned components (A-1), (B-1) and (C-1).

Next, the lipid composition satisfying the abovementioned requirement 2 will be described. The lysophosphatidylcholine (A-2), namely, the component (A) may be obtained by, for example, partially hydrolyzing natural substances such as soybean, rapeseed, corn germ and yolk phospholipids with phospholipase A-2 and fractionating the lysophospholipids thus obtained by solvent fractionation or chromatography. Alternately, synthetic lysophosphatidylcholine or one obtained through biological transesterification of choline phosphatide with various phospholipases (A, B, C or D) mad be employed therefor. Acyl group(s) of the lysophosphatidylcholine (A-2) may be one originating from, for example, linoleic, oleic, linolenic, arachidonic, palmitic, myristic and stearic acids. These acyl groups may bind to either 1- or 2-position of the glycerol. It is preferable that the abovementioned lysophosphatidylcholine (A-2) substantially comprises monoacylphosphatidylcholine. However it may comprise diacylphosphatidylcholine in an amount of 20% by weight or less or a small amount of phosphatides other than choline phosphatide.

When the content of the diacylphosphatidylcholine and other phosphatides in the lysophosphatidylcholine (A-2) exceeds 20% by weight, the solubility of the composition as a whole in water would be undesirably lowered.

Preferable examples of the monoglyceride(s) (B-2) of unsaturated fatty acid(s) having 16 to 22 carbon atoms, namely the component (B), include those carrying at least one unsaturated fatty acid selected from among palmitoleic, oleic, linoleic, linolenic, arachidonic and eicosapentaenoic acids and mixtures of said unsaturated fatty acid(s) with saturated fatty acid(s) as constituting fatty acid(s) and having an iodine value of 50 or above. Those originating from vegetable and/or animal oils falling within the above range may be employed therefor. Further it is preferable that the unsaturated fatty acid monoglyceride(s) (B-2) contain 70% or more or monoester(s). Highly pure monoglycerides such as distilled ones are particularly suitable therefor. These unsaturated fatty acid may bind to the glycerol at any of the 1-, 2- and 3-positions.

An increase in the degree of saturation of the fatty acids constituting said unsaturated fatty acid monoglyceride(s) (B-2) would undesirably lower the solubility of the composition as a whole in water. Unsaturated fatty acids having 15 or less or 23 or more carbon atoms rarely occur in nature.

Examples of the fatty acid(s) (C-2) having 9 to 13 carbon atoms, namely the component (C), include lauric and capric acids. Fatty acids having 8 or less or 14 or more carbon atoms cannot give intense

6

surface activities such as permeability and spreadability. Namely, the main object of the present invention cannot be achieved thereby.

The composition of the present invention comprises the abovementioned lysophosphatidylcholine (A-2), in terms of the pure compound, unsaturated fatty acid monoglyceride(s) (B-2) and fatty acis(s) (C-2) at a ratio by weight satisfying the following relationships:

(B-2)/(A-2) = 10/90 to 90/10,
(C-2)/(B-2) = 20/100 to 200/100, and
(C-2)/((A-2) + (B-2)) = 10/100 to 200/100,

preferably

(B-2)/(A-2) = 20/80 to 80-/20,
(C-2)/(B-2) = 40/199 to 150/100, and
(C-2)/((A-2) + (B-2)) = 25/100 to 140/100.

When the ratio by weight of said unsaturated fatty acid monoglyceride(s) (B-2) to said lysophosphatidylcholine (A-2), in terms of the pure compound, i.e., (B-2)/(A-2), is less than 10/90, the effects of the present invention cannot be satisfactorily achieved. When this ratio exceeds 90/10, on the other hand, the solubility of the composition in water would be undesirably lowered. When the ratio by weight of said fatty acid(s) (C-2) to said unsaturated fatty acid monoglyceride(s) (B-2), i.e., (C-2)/(B-2) is less than 20/100, the effects of the present invention canot be satisfactorily achieved. When this ratio exceeds 200/100, on the other hand, the surface activities cannot be improved and the solubility of the composition in water would be undesirably lowered. When the ratio by weight of said fatty acid(s) (C-2) to the total amount of said lysophosphatidylcholine (A-2) and unsaturatred fatty acid monoglyceride(s) (B-2), i.e., (C-2)/((A-2) + (B-2)) is less than 10/100, the effects of the present invention cannot be satisfactorily achieved. When this ratio exceeds 200/100, on the other hand, the surface activities cannot be improved any more and the solubility of the composition in water would be undesirably lowered.

The composition of the present invention further contains bile acids etc. (D). When the ratio of ((B-2) + (C-2))/(A-2) exceeds approximately 5, in particular, the component (D) should be added.

The same bile acids etc. (D) as those used in the lipid composition satisfying the requirement 1 may be used in this case.

The bile acids etc. (D) may be added in an amount of 5 to 100% by weight based on the total weight of the abovementioned lysophosphatidylcholine (A-2), unsaturated fatty acid monoglyceride(s) (B-2) and fatty acid(s) (C-2). When the amount of the component (D) is less than 5%, the solubilization of the composition cannot be achieved. Also when it exceeds 100%, the solubilization effect cannot be improved any more. Thus it is unnecessary to add such an excessive amount of the bile acids etc. The amount of the bile acids etc. (D) may be appropriately determined within the above range depending on the composition of the components and the kind of the bile acids etc. (D).

Now the lipid composition satisfying the abovemenitoned requirement 3 will be described. Any acylglycerophospholipid (A-3) mad be used as the component (A) without restriction, so long as it contains at least 10% by weight of monoacylglycerophospholipid(s) and substantially comprises a mixture of mono- and diacylglycerophospholipids. Similar to the case of the acylglycerophospholipid(s) (A-1) in the lipid composition satisfying the requirement 1, partially hydrolyzed acylglycerophospholipids may be employed therefor. These partially deacylated acylglycero-phospholipids may be further fractionated by, for example, using a solvent such as an aqueous alcohol optionally together with a silica gel column to thereby elevate the content of mono- and diacylglycerophospholipids.

The abovementioned acylglycerophospholipid(s) (A-3) may be a mixture of various phosphatides having mono or diacyl groups. For example, a mixture of monoacyl and/or diacylphosphatidylcholine, phosphatldylethanolamine, phosphatidylinositol, phosphatidic acid and phosphatidylserine may be used therefor. Furthermore, mono- and diacylglycerophospholipids whose constitutents such as acyl groups or choline are chemically modified with the use of phospholipases A-1, A-2, or A1, A2, B or D may be used as the acylglycerophospholipid(s) (A-3).

It is preferable that said acylglycerophospholipid(s) (A-3) contain the mono acyl component at a ratio of at least 10% by weight, still preferably at least 25% by weight, based on the total amount of the monoacyl and diacyl components. When the content of the monoacyl component is less than 10% by weight based on the total amount of the monoacyl and diacyl components, the effects of the present invention can be hardly achieved. The upper limit of the content of the monoacyl component is not particularly specified,

7

though a content thereof exceeding 80% by weight cannot obviously improve the effects of the invention and more. However acylglycerophospholipid(s) containing 80 to 100% by weight of the monoacyl component is also available in the present invention, though it is expensive.

The abovemenitoned acylglycerophospholipid(s) (A-3) containing at least 10% by weight of the mono acyl component may substantially comprises mono- and diacylphosphatidylcholines. It is preferable that the content of the monoacylphosphatidylcholine is at least 10% by weight, still preferably at least 20% by weight, based on the total amount of the mono and diacyl components. The upper limit of the content of the monoacylphosphatidylcholine is not particularly specified. Thus highly pure monoacylphosphatidylcholine may be used as said acylglycerophospholipid (A-3). However the use of 80% by weight or more of monoacylphosphatidylcholine cannot give any remarkable increase in the surface activities any more. Further, such a composition is disadvantageous from the economic viewpoint, since it is inferior in surface activities to those comprising phospholipids other than choline phospholipids. Furthermore, such a composition shows an unsatisfactory solubilization effect on fatty acids and fatty acid monoglycerides.

Examples of the phospholipids available as the starting material in the preparation of the abovementioned acylglycerophospholipid(s) (A-3) include soybean, rapeseed and yolk phospholipids. In addition, other phospholipids may be used therefor and those lacking some components other than phosphatidylcholine are also available.

The abovementioned acylglycerophospholipid(s) (A-3) may be analyzed by various methods. For example, the component (A-3) may be subjected to thin layer chromatography and examined on the spots thus formed. Alternately, it may be analyzed with a TLC-FID analyzer (Iatro Scan method), by high performance liquid chromatography or by combining these procedures.

The monoglyceride(s) (B-3) of medium-chain fatty acid(s) having 8 to 13 carbon atoms, namely the component (B), may be selected from, for example, natural ones, fats or oils decomposed by lipase or subjected to glycerolysis and synthetic ones. Those chemically synthesized from fatty acids originating in natural substances, partially hydrogenated fatty acids, distilled or solvent-fractionated fatty acids and glycerol are commonly employed therefor.

The abovementioned medium-chain fatty acid monoglyceride(s) (B-3) may be either 1-, 3-glycerides or 2-glycerides with respect to the carbon atoms of the glycerol.

The abovementioned medium-chain fatty acid monoglyceride(s) (B-3) comprises glycerides of fatty acids having 8 to 13 carbon atoms. Preferable examples thereof are medium-chain fatty acid monoglycerides containing at least 40% by weight of monoesters and comprising saturated fatty acids exclusively or a mixture of saturated and unsaturated fatty acids as the constituting fatty acids. When the monoester content of the medium-chain fatty acid monoglyceride(s) is less than 40% by weight, the effects of the present invention cannot be satisfactorily achieved. Further those containing 70% by weight or more monoester(s) are preferable since stable effects can be achieved thereby. Furthermore, distilled monoglycerides are still preferable.

Examples of the fatty acids constituting the abovementioned medium-chain fatty acid monoglyceride(s) include saturated fatty acids such as caprylic, capric, lauric, undecanoic and tridecanoic acids and unsaturated fatty acids such as obtusilic, linderic and lauroleic acids.

When monoglycerides of fatty acids having seven or less carbon atoms are employed, the improved surface activities including permeability and wettability, namely, the object of the present invention cannot be achieved. In addition, such a composition would show an intense offensive odor when an aqueous micelle solution of the same is, even slightly, hydrolyzed. Also when monoglycerides of fatty acids having 14 or more carbon atoms, in particular saturated fatty acids, are employed, the improved surface activities including permeability and wettability, namely, the object of the present invention cannot be achieved.

The fatty acid(s) (C-3), namely the component (C),may comprise saturated fatty acid(s) having 8 to 18 carbon atoms and/or unsaturated fatty acid(s) having 14 to 22 carbon atoms. When saturated fatty acids having seven or less or 19 or more carbon atoms are employed as the fatty acids (C-3), the improved surface activities including permeability and wettability, namely, the object of the present invention cannot be achieved. Further, a fatty acid having seven or less carbon atoms is undesirable since it has an intense offensive odor. Particularly preferable examples of the fatty acid(s) (C-3) are saturated fatty acids having 10 to 14 carbon atoms.

Examples of the unsaturated fatty acids having 14 to 22 carbon atoms to be used as the abovementioned fatty acid(s) (C-3) include palmitoleic, oleic, linoleic, linolenic, arachidonic, eicosapentaenoic and docosahexaenoic acids. Generally speaking, the surface activities including permeability and wettability of the composition can be elevated with an increase in the degree of unsaturation (iodine value) of the unsaturated fatty acid(s). However the effects of these unsaturated fatty acids are somewhat lower than those achieved by the abovementioned saturated fatty acids having 10 to 14 carbon atoms. Said unsaturated fatty acids are superior to the saturated ones, however, in the emulsifying and dispersing effects. Unsaturated fatty acids having 13 or less or 23 or more carbon atoms are inavailable in practice since they hardly occur in nature.

Either one of these saturated and unsaturated fatty acids or a mixture thereof may be employed as the component (C-3). A mixture of medium-chain fatty acid(s) obtained from, for example, coconut oil or palm kernel oil with short- and long-chain fatty acid(s) is a preferable example of the abovementioned fatty acid(s) (C-3).

The composition of the present invention comprises the abovementioned acylglycerophosphollpid(s) (A-3), medium-chain fatty acid monoglyceride(s) (B-3) and fatty acid(s) (C-3) at a ratio by weight satisfying the following relationships:

(B-3)/(A-3) = 10/90 to 95/5,
(C-3)/(B-3) = 20/100 to 250/100, and
(C-3)/((A-3) + (B-3)) = 10/100 to 200/100,

preferably

(B-3)/(A-3) = 30/70 to 90/10,
(C-3)/(B-3) = 30/100 to 170/100, and
(C-3)/((A-3) + (B-3)) = 25/100 to 150/100.

When the ratio by weight of said medium-chain fatty acid monoglyceride(s) (B-3) to said acylglycerophospholipid(s) (A-3), i.e., (B-3)/(A-3) is less than 10/90, the effects of the present invention cannot be satisfactorily achieved. When this ratio exceeds 95/5, on the other hand, the solubility of the composition in water would be undesirable lowered. When the ratio by weight of said fatty acid(s) (C-3) to said medium-chain fatty acid monoglyceride(s) (B-3), i.e., (C-3)/(B-3) is less than 20/100, the effects of the present invention cannot be satisfactorily achieved. Also when this ratio exceeds 250/100, the effects would not be improved any more. Furthermore the solubility of the composition in water would be undesirably lowered in this case.

When the ratio by weight of said fatty acid(s) (C-3) to the total amount of said acylglycerophospholipid-(s) (A-3) and medium-chain fatty acid monoglyceride(s) (B-3), i.e., (C-3)/((A-3) + (B-3)) is less than 10/100, the effects of the present invention cannot be satisfactorily achieved. Also when this ratio exceeds 200/100, the effects would not be improved any more. Furthermore, the solubility of the composition in water would be undesirably lowered in this case.

When the ratio by weight of ((B-3) + (C-3))/(A-3) exceeds 6, the composition of the present invention should contain bile acids etc. (D) in an amount of 5 to 100% by weight based on the total amount of the components (A-3), (B-3) and (C-3).

The bile acids etc. (D) mad be the same as those used in the lipid composition satisfying the requirement 1.

When the composition of the present invention contains a small amount of monoacylglycerophospholipid(s) in the abovementioned acylglycerophospoholipid(s) (A-3) and a small amount of monoglyceride(s) in the abovementioned medium-chain fatty acid monoglyceride(s) (B-3), it is preferable to add the bile acids etc. (D) thereto.

The addition of the bile acids etc. (D) enables stable solubilization of said medium-chain fatty acid monoglyceride(s) (B-3) and fatty acid(s) (C-3), even if the content of monoacylglycerophospholipid(s) in said acylglycerophospholipid(s) (A-3) is relatively low.

The upper limit of the bile acids etc. (D) to be added is not particularly specified. However the effects cannot be improved any more when the component (D) is added in an amount exceeding the total amount of the abovementioned acylglycerophospholipid(s) (A-3), medium-chain fatty acid monoglyceride(s) (B-3) and fatty acid(s) (C-3). Therefore this may be the upper limit of the content of the bile acids etc. (D) from the economic viewpoint. On the other hand, the aimed effects can be achieved when at least 5% by weight of the bile acids etc. (D) are added, based on the total amount of the abovementioned acylglycerophospholipid(s) (A-3), medium-chain fatty acid monoglyceride(s) (B-3) and fatty acid(s) (C-3). Thus it may be the lower limit of the same.

The addition of the abovementioned bile acids etc. (D) causes little changes in the surface tension lowering effect and contact angle of the composition. The wetting time determined by the canvas disc method seems to be shortened thereby, though this effect could be hardly enhanced when the bile acids etc. (D) are added in an amount exceeding 50% by weight of the total amount of the abovementioned components (A-3), (B-3) and (C-3).

A lipid composition of the present invention satisfying the above requirement 1, 2 or 3 is liable to be affected by microorganisms or oxidation. Therefore it may be preferably stabilized by an appropriate method such as sterilization or addition of an antioxidant.

A micelle solution obtained by dissolving a lipid composition satisfying the abovementioned requirement 1, 2 or 3 shows a high permeability and a high wettability. Further it shows a high surface tension lowering effect.

A lipid composition satisfying the abovementioned requirement 1, 2 or 3 may be produced by, for example, the following methods.

I. Lipid composition of the present invention satisfying the abovementioned requirement 1:

(1) When fatty acid monoglyceride(s) (B-1) and fatty acid(s) (C-1) are used each in a relatively large amount, acylglycerophospholipid(s) (A-1) are added to the fatty acid monoglyceride(s) (B-1) and fatty acid(s) (C-1). When bile acids etc. (D) are further employed, the component (D) is added together with the acylglycerophospholipid(s) (A-1) thereto. The resulting mixture is molten by heating to thereby give the composition of the present invention.

(2) When acylglycerophospholipid(s) (A-1) are used in a relatively large amount and when said acylglycerophospholipid(s) (A-1) contains a large amount of monoacylglycerophospholipid(s), these components are molten together by heating with the use of a small amount of a solvent such as hexane or ethanol. Then the solvent is removed under reduced pressure and the remaining mixture is formulated into a paste or granules to thereby give the composition of the present invention. Alternately, the mixture may be dissolved or dispersed in water. Thus the composition of the present invention can be obtained in the form of an aqueous solution. Furthermore, the aqueous dispersion may be concentrated under reduced pressure to thereby give the composition of the present invention in the form of an aqueous paste.

(3) When acylglycerophospholipid(s) (A-1) contains a relatively large amount of monoacylglycerophospholipid(s), fatty acid(s) (C-1) are used in a relatively small amount and bile acids etc. (D) are used in a relatively large amount, these components are dispersed in water to thereby give the composition of the present invention in the form of an aqueous solution. Further, this aqueous dispersion may be concentrated under reduced pressure to thereby give the composition of the present invention in the form of an aqueous paste. When the composition is highly soluble in water as a whole and all of the components are present in the form of solids at room temperature, the composition may be formulated into a powder, small particles or pellets.

(4) When the composition of the present invention is to be prepared in the form of an aqueous colloidal or micelle solution of a concentration suitable for the final use, these components are added to water and the obtained mixture is treated with a homogenizer under heating or is ultrasonically treated. Thus the composition of the present invention in the form of an aqueous colloid or micellar solution can be readily prepared.

(5) Another method for preparing the composition of the present invention comprises hydrolyzing one part by weight of commercially available phospholipids containing oil or fat, such as soybean phospholipids, by adding 0.1 to 1 part by weight of water and pancreatic phospholipase A-2 at 50 to 60°C. After hydrolysis of phospholipids, water is further added thereto followed by the addition of the enzyme having a site-specificity for glyceride, such as pancreatic lipase. Mucor lipase or Rhizopus lipase and the obtained mixture is maintained at 40°C. Thus the oil (triglyceride) is hydrolyzed Into monoglycerides and fatty acids to thereby give the composition of the present invention.

The abovementioned acylglycerophospholipid(s) (A-1) constituting the composition of the present invention satisfying the abovementioned requirement 1 have no such high surface activities per se. The abovementioned fatty acid monoglyceride(s) (B-1) are hardly soluble in water and have scarcely any surface activities other than an emulsifying effect. Furthermore, the fatty acid(s) (C-1) are substantially insoluble in water and have almost no surface activities. Namely, each component of the composition of the present invention satisfying the requirement 1 has insufficient activities. However intense surface activities can be obtained when these three components are present together.

The addition of the abovementioned bile acids etc. (D), particularly alkali salt(s) of bile acids, to the composition could give little effect from the viewpoint of the surface activities. Since, however, the component (D) can solubilize fatty acids and fatty acid monoglycerides which are inherently insoluble or hardly soluble in water, the addition of the component (D) to a composition which is hardly soluble in water per se can induce the surface activities.

II. Lipid composition satisfying the abovementioned requirement 2:

(1) When unsaturated fatty acid monoglyceride(s) (B-2) and fatty acid(s) (C-2) are used each in a relatively large amount and these components are used together with lysophosphatidylcholine (A-2) and bile acids etc. (D), all of the components are mixed and melted together by heating to thereby give the composition of the present invention in the form of a viscous oily paste.

(2) When lysophosphatidylcholine (A-2) is used in a relatively large amount and the total amount of unsaturated fatty acid monoglyceride(s) (B-2) and lysophosphatidylcholine (A-2) is relatively large, all of the components are dissolved in water in order of solubility optionally together with bile acids etc. (D), if required. The solution thus obtained may be used as such as the composition of the present invention. Alternately, each component is simply added to water and the obtained solution is heated and formulated into an aqueous colloidal solution by, for example, an ultrasonic treatment. It is further possible to prepare the composition of the present invention in the form of an aqueous solution by using a mixer provided with rotary blades or a pressure homogenizer. Furthermore, the water may be removed from said aqueous solution under reduced pressure to thereby give the composition in the form of an aqueous paste.

(3) The composition of the present invention may be readily obtained by adding each component to a solvent mixture comprising, for example, hexane, ethanol and ether and dissolved therein by heating. Then the solvent is removed from the obtained solution under reduced pressure. Thus the aimed composition can be obtained in the form of a paste.

The abovementioned bile acids etc. (D), namely, the fourth component of the composition of the present invention satisfying the above requirement 2, can exert little effect of improving the surface activities per se. However the component (D) promotes the solubilization of the composition. Further, its addition somewhat decrease the surface tension lowering effect of the aqueous solution of the composition, shows little effect on the contact angle and somewhat elevates the permeability.

Among the components of the composition of the present invention satisfying the abovementioned requirement 2, namely, said lysophosphatidylcholine (A-2), unsaturated fatty acid monoglyceride(s) (B-2) and fatty acid(s) (C-2), the lysophosphatidylcholine alone is soluble in water and thus has permeation and wetting effects. Namely, the two other components, which are substantially insoluble in water, are solubilized by the lysophosphatidylcholine.

A two-component composition comprising the lysophosphatidylcholine (A-2) and unsaturated fatty acid monoglyceride(s) (B-2) shows no or little improvement in the permeability or wettability, compared with those of lysophosphatidylcholine per se of the same concentration. However the addition of medium-chain fatty acid(s) (C-2) having 9 to 13 carbon atoms to this system remarkably improves the surface activities, as observed in the case of the composition of the present invention.

III. Lipid composition of the present invention satisfying the abovementioned requirement 3:

(1) When medium-chain fatty acid monoglyceride(s) (B-3) and fatty acid(s) (C-3) are used each in a relatively large amount, acylglycerophospholipid(s) (A-3) are added, optionally together with bile acids etc. (D), to the medium-chain fatty acid monoglyceride(s) (B-3) and fatty acid(s) (C-3). The obtained mixture is melted by heating to thereby give the composition of the present invention .

(2) When acylglycerophospholipid(s) (A-3) are used in a relatively large amount or when said acylglycerophospholipid(s) (A-3) contain a large amount of monoacylglycerophospholipid(s), these components are melted together by using a small amount of a solvent such as hexane, ethanol or ether by heating. Then the solvent is removed under reduced pressure and the obtained mixture is formulated into a paste or particles. Thus the composition of the present invention is obtained. Alternately, said mixture may be dissolved or dispersed in water to thereby give the composition of the present invention in the form of an aqueous solution. Furthermore, the aqueous dispersion may be concentrated under reduced pressure to thereby give the composition of the present invention in the form of an aqueous paste.

(3) When acylglycerophospholipid(s) (A-3) contain a large amount of monoacylglycerophospholipid(s), when fatty acid(s) (C-3) is a composition of medium-chain fatty acids, or when bile acids etc. (D) are used in a relatively large amount, these components are dispersed or dissolved in water by heating to thereby give the composition of the present invention in the form of an aqueous solution. The aqueous solution or dispersion may be concentrated under reduced pressure to thereby give the composition of the present invention in the form of an aqueous paste. In these cases, the composition may be formulated into a powder, small particles or pellets, when the composition is highly soluble in water as a whole and all of the components are present in the form of a solid at room temperature.

(4) In order to prepare the composition of the present invention in the form of an aqueous colloidal or micelle solution of a concentration suitable for the final use, these components are added to water followed by treating with a homogenizer by heating or ultrasonic treating. Thus an aqueous colloidal or micelle solution of the composition of the present invention can be readily prepared.

(5) Another method for preparing the composition of the present invention comprises hydrolyzing one part by weight of commercially available phospholipids containing fats or oils, such as soybean phospholipids, by adding 0.1 to 1 part by weight of water and pancreatic phospholipase A-2 at 50 to 60°C. After hydrolysis of phospholipids, water is further added thereto followed by an addition of enzyme having a 1-or 3-position site-specificity for glyceride, such as pacreatic lipase. Mucor lipase or Rhizopus lipase and the obtained mixture is maintained at approximately 40°C. Thus the oil (triglycerides) is decomposed into monoglycerides and fatty acids to thereby give the composition of the present invention.

The abovementioned acylglycerophospholipid(s) (A-3) constituting the composition of the present invention satisfying the above requirement 3 have no such high surface activities by themselves. The abovementioned medium-chain fatty acid monoglyceride(s) (B-3) having 8 to 10 carbon atoms show high permeability and wettability immediately after being solubilized by heating together with water. However the resulting aqueous solution is unstable at room temperature and the monoglycerides would be precipitated with the lapse of time. Thus the surface activities are lost. All of the abovementioned fatty acid(s) (C-3) are substantially insoluble in water. Namely, each component of the composition of the present invention has insufficient surface activities. However intense surface activities exceeding those of conventional industrial surfactants can be achieved when these three components are used together.

The addition of the bile acids etc. (D), in particular, alkali salts of bile acids to the composition of the present invention would give little effect regarding the surface activities. However the component (D) would solubilize fatty acids and fatty acid monoglycerides which are inherently insoluble or hardly soluble in water. Therefore the addition of the component (D) to a composition which is hardly soluble in water per se can induce the surface activities.

The lipid composition of the present invention satisfying the abovementioned requirement 1, 2 or 3 may further comprise other surfactants so long as the achievement of the object of the present invention is not inhibited thereby. However care should be taken since the addition of a large amount of other surfactants might deteriorate the effects of the composition of the present invention. Furthermore, the composition of the present invention may contain a small amount of cholesterol, sphingolipids or glycolipids as well as some impurities such as fatty acid triglycerides or diglycerides, so long as the effects of the present invention are not deteriorated thereby.

The composition of the present invention satisfying the abovementioned requirement 1, 2 or 3 is characterized in that it exclusively consists of the constituents of living organisms and thus has a high safety.

The lipid composition of the present invention satisfying the abovementioned requirement 1, 2 or 3 is available as a penetrating agent, a wetting agent and a spreading agent. In addition, it may be used as a dispersant for insoluble microparticles, an oil-in-water type emulsifier, a water-in-oil type emulsifier and a solubilizer of a hydrophilic substance in an oily phase.

The lipid composition of the present invention satisfying the abovementioned requirement 1, 2 or 3 containing a relatively large amount of the monoacylglycerophospholipid(s) (A) or bile acids etc. (D) would form micelles in water to thereby give a stable dispersion. When said composition contains a relative large amount of the fatty acid(s) (C) or fatty acid monoglycerides(s) (B), the dispersion would suffer from the precipitation of these microcrystals when allowed to stand. However the precipitate can be readily redispersed by heating. Therefore the composition of the present invention may be readily introduced into cereal doughs in the preparation of, for example, bread, udon noodles, soba noodles, macaroni or spaghetti.

The lipid composition of the present invention satisfying the abovementioned requirement 1, 2 or 3 has a high safety and intense surface activities. Thus it is useful as a penetrating and/or wetting agent in the improvement of the wettability of powdery foods, feeds, cosmetics or drugs; as a rinse for hair or dishes; as a spreading agent (sticker) of agricultural chemicals for plant foliage or insects; or in the improvement of wettability of fiber products such as fabrics or paper. More particularly, it is applicable to oil-in-water type of water-in-oil type emulsification or solubilization of edible fats and oils, vegetable essential oils, paraffins and other fat materials; dispersion, wetting into water or promotion of the dispersion of various powdery materials such as cocoa powder, powdery instant foods, powdery spices, antifungal agent such as p-hydroxybenzoic acid and various powdery pigments; emulsification or dispersion of these materials under somewhat acidic conditions; and solubilization of insoluble materials in fats and oils.

12

Further, the lipid composition of the present invention satisfying the abovementioned requirement 1, 2 or 3 would separate proteins from, for example, animal or plant sell membranes. In this field, polyoxyethylene nonylphenol ether is often used as an intense surfactant, though it would denature some proteins. Alternately, lysophosphatidylcholine may be used for this purpose, though it is extremely expensive. In contrast thereto, the composition of the present invention is highly safe and effective and yet inexpensive.

Furthermore, it is to be emphasized here that the high permeability of the lysophosphatidylcholine composition of the present invention satisfying the abovementioned requirement 1, 2 or 3 makes it highly effective in the promotion of the transintestinal, subcutaneous and transmucosal absorption of drugs. It is also possible to apply the composition of the present invention per se as a drug by incorporating physiologically active polyunsaturated fatty acids such as eicosapentaenoic or $\gamma$-linolenic acid or odd-number acids such as pentadecanoic acid. Furthermore, the lipid composition of the present invention satisfying the abovementioned requirement 1, 2 or 3 may be applied to a fat emulsion together with other nutrients to thereby give a highly digestive transintestinal nutritional agent. The effect of the composition of the present invention in accelerating the absorption of drugs may be applied not only to mammals but also to insects and plants including crops.

The lipid composition of the present invention satisfying the abovementioned requirement 1, 2 or 3 is highly soluble either in water or in a nonpolar solvent such as an oil and form micelles. Namely, it is an amphipathic material.

To further illustrate the present invention, and not by way of limitation, the following Examples, Referential Examples relating to the production of the acylglycerophospholipids to be used in the present invention, and Comparative Examples will be given.

Unless otherwise noted, all percentages and parts are by weight.

Referential Example 1

14 parts of water was added to 100 parts of commercially available soybean phopholipids (Soybean lecithin; mfd. by Ajinomoto Co., Inc.). Further 0.25 part of phospholipase A-2 (Lecithase 10-L; mfd. by Novo) was added thereto and the resulting mixture was stirred. Then it was allowed to stand at 50° to 55°C for 0.5, 1, 3, 15 and 36 hours to thereby monoacylate the same. Each reaction mixture was dehydrated under reduced pressure and centrifuged to thereby remove most of triglycerides and fatty acids. Subsequently it was treated with acetone to thereby remove remaining impurities. An insoluble residue was dried under reduced pressure. Thus acylglycerophospholipids (1) to (5) were obtained. Each of the acylglycerophospholipids will be simply referred to as the phosphatide hereinafter.

Table 1 shows the content (% by weight) of the monoacyl component based on the total amount of the mono- and diacyl components of each phosphatide thus obtained.

Table 1

| Phosphatide No. | Enzymatic treatment (hr) | Monoacyl content (%) |
|---|---|---|
| (1) | 0.5 | 18 |
| (2) | 1 | 33 |
| (3) | 3 | 51 |
| (4) | 15 | 66 |
| (5) | 36 | 82 |

Referential Example 2

Commercially available yolk phospholipids (mfd. by Asahi Chemical Industry Co., Ltd.; purity of 96%) were ultrasonically dispersed in water and treated with phospholipase A-2 for 40 hours, similar to the procedure of Referential Example 1. After treating with acetone to thereby remove impurities, phosphatide (6) were obtained.

This phosphatide (6) contained 86% by weight of the monoacyl component based on the total amount of the mono- and dicomponents.

Referential Example 3

Commercially available soybean phospholipids were defatted with acetone and fractionated with an aqueous alcohol. Thus phosphatide containing 70% of phosphatidylcholine was obtained. After treating with phospholipase A-2 (Lecithase 10-L; mfd. by Novo), the phosphatide was treated with acetone to thereby remove fatty acids. Then it was further treated with an aqueous alcohol and subjected to silica gel chromatography with the use of an aqueous alcohol. Thus phosphatide (7) containing 97% of phosphatidylcholine was obtained.

Each of 0.5% solutions of these phosphatides (1) to (7) obtained in the above Referential Examples 1 to 3 was examined by the canvas disc method (cf. Kaimen Kasseizai Binran , p. 860, Sangyo Tosho K.K. (1960)). The permeabilities (measured by the sedimentation time of canvas disk: canvas disk wetting time) immersed in aqueous solution, of these phosphatides thus determined were as follows:

| phosphatide (1) | 6.3 min.; |
| phosphatide (2) | 10.0 min.; |
| phosphatide (3) | 6.1 min.; |
| phosphatide (4) | 3.0 min.; |
| phosphatide (5) | 1.6 min.; |
| phosphatide (6) | 1.5 min.; and |
| phosphatide (7) | 1.2 min. |

The surface tensiodn of each of 0.5% solutions of the phosphatides (1) to (7) obtained in the above Referential Examples 1 to 3 was measured by a surface tensiodn meter (CBVP A-3, mfd. by Kyowa Kagaku K.K. Tokyo Japan) with a platinum plate at 35°C. The results were as follows:

| phosphatide (1) | 35.7 dynes/cm; |
| phosphatide (2) | 33.4 dynes/cm; |
| phosphatide (3) | 31.8 dynes/cm; |
| phosphatide (4) | 31.4 dynes/cm; |
| phosphatide (5) | 31.1 dynes/cm; |
| phosphatide (6) | 29.7 dynes/cm; and |
| phosphatide (7) | 37.2 dynes/cm. |

Furthermore, the contact angles on bees wax and on Japan wax at 30°C of each of 0.5% solutions of the phosphatides (1) to (5) obtained in the above Referential Example 1 were measured with a contact angle meter (Elmer-13: mfd. by Elmer Kogaku K.K. Tokyo Japan). Table 2 shows the results.

Table 2

| | (degrees) | | | | |
|---|---|---|---|---|---|
| | (1) | (2) | (3) | (4) | (5) |
| Bees wax | 71° | 67° | 57° | 54° | 48° |
| Japan wax | 84° | 69° | 52° | 48° | 44° |

The following commercially available fatty acid monoglycerides were employed in the following Examples and Comparative Examples.

Emulsy MU: (unsaturated fatty acid monoglycerides) Linoleic acid monoglycerides ($C_{16}$ to $C_{20}$) Containing 95% of monoester, 1% of diester and no triester.
Iodine value: 116.

Emulsy OL: (unsaturated fatty acid monoglycerides) Oleic acid monoglycerides ($C_{14}$ to $C_{20}$).
Containing 93% of monoester, 1% of diester and no triester.
Iodine value: 67.

Emulsy MTT; (unsaturated fatty acid monoglycerides) Beef tallow fatty acid mon-

14

oglycerides ($C_{14}$ to $C_{20}$)

Containing 90% of monoester, 7% of diester and no triester.

Iodine value: 36.

Poem CS-200: (unsaturated fatty acid monoglycerides) Cotton seed oil mono and diglycerides ($C_{14}$ to $C_{20}$).

Containing 45% of monoester, 43% of diester and 12% of triester.

Iodine value:100.

Emulsy MS: (saturated fatty acid monoglycerides) Hardened beef tallow monoglycerides (64% of $C_{18}$ and 30% of $C_{16}$).

Containing 98% of monoester.

Iodine value: 1.6.

Poem C-100: (medium-chain fatty acid monoglycerides) Hardened coconut oil monoglycerides ($C_6$ to $C_{18}$).

Containing 86% of monoester, 10% of diester and 0.4% of triester.

Iodine value: 2.2

Constituting fatty acids: $C_6$ : 0.3%;

$C_8$ : 7%;

$C_{10}$ : 6%

$C_{12}$ : 51%

$C_{16}$ : 8%;

$C_{18}$ : 8%;

monounsaturated $C_{18}$ : 2%; and

diunsaturated $C_{18}$ : 0.4%.

Poem M-300: (medium-chain fatty acid monoglycerides) Lauric acid monoglycerides.

Containing 84% of monoester, 10% of diester and 0.6% of triester.

Iodine value: 1.7

Constituting fatty acid: $C_{12}$ : 98%.

Poem M-200: (medium-chain fatty acid monoglycerides) Capric acid monoglycerides.

Containing 92% of monoester, 4% of diester and no triester.

Iodine value: 1.8.

Constituting fatty acids: $C_{10}$ : 98%.

Poem M-100: (medium-chain fatty acid monoglycerides) Caprylic acid monoglycerides.

Containing 86% of monoester, 9% of diester and no triester.

Iodine value: 1.7.

Constituting fatty acids: $C_8$ : 97%.

The fatty acids employed in the following Examples were the following commercially available products.

Linoleic acid: Mfd. by Asahi Denka Kogyo K.K.

Distilled product.

Purity: 99% or above.

Iodine value: 179.

Oleic acid: Mfd. by Asahi Denka Kogyo K.K.

Distilled product.

Purity: 99% or above.

Iodine value: 89.

Stearic acid: Mfd. by Nippon Oils and Fats Co., Ltd.

NAA-180.

Purity: 96% or above.

Palmitic acid: Obtained by distilling PA-900 (mfd. by Asahi Denka Kogyo K.K.).

Purity: 99% or above.

Myristic acid $C_{14}$: Mfd. by Nippon Oils and Fats Co., Ltd.

NAA-142

Purity: 99% or above.

Coconut oil fatty acid: Mfd. by Nippon Oils and Fats Co., Ltd.

Lauric acid $C_{12}$: Mfd. by Nippon Oils and Fats Co., Ltd.

NAA-122

$C_{12}$ acid: 99%

Capric acid $C_{10}$: Mfd. by Kao Corp.

Lunac 10-95

| | C$_{10}$ acid: 95%. |
|---|---|
| Caprylic acid C$_8$: | Mfd. by Kao Corp. |
| | Lunac 8-95 |
| | C$_8$ acid: 95%. |
| Caproic acid C$_6$: | Obtained by distilling a first-grade reagent (mfd. by Kanto Kagaku K.K.). |
| | C$_6$ acid: 99%. |

The bile acids etc. employed in the following Examples were as follows.

| | |
|---|---|
| Sodium taurocholate: | Mfd. by Difco Lab. |
| | Purity: 70%. |
| Glycocholic acid: | Mfd. by Sigma. |
| | Crystalline. |
| Sodium glycocholate: | Mfd. by Sigma. |
| | Synthetic. |
| | Purity: 99%. |
| Cholic acid: | Mfd. by Wako Pure Chemicals Co. |
| | Purity: 98% or above. |
| Sodium cholate: | Mfd. by Wako Pure Chemicals Co. |
| | Purity: 98.5% or above. |

In the following Examples and Comparative Examples, the surface activities and spreadability of each sample were examined each in the following manner.

Test on surface activities:

Water was added to a test composition to thereby give micelle solutions of solid (except bile acids etc.) concentrations of 1.0, 0.8, 0.5 and 0.2%. The permeability of each solution thus obtained was measured at 37°C, or 25°C in some cases, by the canvas disc method in the same manner as the one described regarding the phosphatides obtained in Referential Examples 1 to 3. Further the surface tensiodn of each sample was measured with a surface tension meter CBVP A-3 (mfd. by Kyowa Kagaku K.K.) at 35°C. Furthermore, the contact angles on bees wax and Japan wax of each sample were measured at 30°C with a contact angle meter Elmer-13 (mfd. by Elmer Kogaku K.K.) at 30°C.

Test on spreadability on foliage:

An aqueous spreading solution comprising 0.1% by volume, in terms of solid content except the bile acids etc., of each composition to be tested, 0.06% by volume of xanthan gum (Echo Gum; mfd. by Merck) and a small amount of food dye blue No. 1 (Brilliant Blue FCF; mfd. by Sanei Kagaku Kogyo K.K.) was subjected to the following spreading test.

Test pieces (10 x 6 cm) were prepared by using sixth to eighth, from the outside, cabbage leaves to which stipes had been avoided. A test piece was immersed in 300 ml of a spreading solution at 25°C for 10 seconds and then air-dried on a filter paper. Thus the spreading of the blue dye on the leaf piece was observed and evaluated according to the following criteria, as a scores from 5 to one:

5: 90% or more of the leaf surface was colored;
4: 70 to 90% of the leaf surface was colored;
3: 50 to 70% of the leaf surface was colored;
2: 30 to 50% of the leaf surface was colored; and
1: 10 to 30% of the leaf surface was colored.

First, Examples for the lipid composition of the present invention satisfying the abovementioned requirement 1 will be given.

Example 1

Seven parts of the phosphatide (7), 36 parts of Emulsy MU, 57 parts of linoleic acid and 36 parts of sodium taurocholate were dispersed in 2000 parts of water by heating. The resulting dispersion was homogenized with the high-pressure homogenizer 3205-200 LH type (mfd. by Sanwa Kikai K.K.) at 60°C under a valve pressure in the first step of 150 kg/cm$^2$ and that in the second step of 50 kg/cm$^2$.

The micelle solution thus obtained was concentrated to thereby give 500 parts of a composition in the form of an aqueous paste.

390 g of water was added to 10 g of this composition. Thus a micelle solution having a solid concentration, except for the sodium taurocholate, of 0.5% was obtained. This micelle solution was subjected to the abovementioned test on surface activities.

As a result, this composition showed a wetting time of 15.2 sec., a surface tension of 28.1 dynes/cm and a contact angle on bees wax of 33° in the canvas disc method at 37°C.

Example 2

21 g of the phosphatide (5), 30 g of sodium taurocholate, 30 g of Emulsy MU and 49 g of linoleic acid were dissolved and/or dispersed in 370 g of water heated to 60°C in this order by using a TK homogenizer HV-M type (mfd. by Tokushu Kika Kogyo K.K.). Thus 500 g of a composition was obtained.

Water was added to 10 g of this composition to thereby give a total amount of 400 g. Thus an aqueous solution having a solid concentration, except for the sodium taurocholate, of 0.5% was obtained. The surface activities of this aqueous solution was examined in the same manner as the one described in Example 1.

As a result, this composition showed a wetting time of 14.5 sec., a surface tensidon of 27.9 dynes/cm a contact angle on bees wax of 37° degrees. Another aqueous solution having a solid concentration, other than the sodium taurocholate, showed a permeation period of 39.7 sec.

Example 3

Water was added to 280 mg of the phosphatide (5), 280 mg of Emulsy MU and 440 mg of linoleic acid to thereby give a total amount of 200 g. The resulting mixture was heated to 50°C and dissolved by using an ultrasonic vibrator UD-200 (mfd. by Tomy Seiko K.K.). Thus a cloudy micelle solution was obtained.

The surface activities of this micellar solution were examined in the same manner as the one described in Example 1.

As a result, this composition showed a wetting time of 19.4 sec., a surface tension of 27.8 dynes/cm and a contact angle on bees wax of 37° degrees.

Example 4

The procedure of Example 2 was repeated to thereby give a composition comprising 19 g of the phosphatide (3), 45 g of Emulsy MU, 36 g of linoleic acid, 13 g of sodium taurocholate and 387 g of water.

7.8 g of this composition was dissolved in water to thereby give a total amount of 200 g. Thus an aqueous solution having a solid concentration, except for the sodium taurocholate, of 0.78% was obtained. The surface activities of this aqueous solution were measured in the same manner as the one described in Example 1.

As a result, this composition showed a wetting time of 10.2 sec., a surface tension of 28.4 dynes/cm and a contact angle on bees wax of 36° degrees.

Example 5

48 parts of the phosphatide (3), 20 parts of Emulsy OL. 32 parts of oleic acid and 10 parts of sodium taurocholate were mixed together to thereby give a total amount of 3.3 g. Then water was further added thereto to thereby give a total amount of 300 g. The obtained mixture was heated to 50°C and dissolved and/or dispersed by using an ultrasonic vibrator UD-200 (mfd. by Tomy Seiko K.K.). The surface activities of the aqueous solution thus obtained, which had a solid concentration, except for the sodium taurocholate, of 1%, were examined in the same manner as the one described in Example 1.

As a reslt, this composition showed a wetting time of 22.9 sec., a surface tension of 26.9 dynes/cm and a contact angle on bees wax of 34° degrees. Furthermore, the spreadability of this composition on foliage was examined by the abovementioned method. As a result, the spreadability thereof was evaluated to be 3 as scour.

Example 6

The phosphatide (6) (yolk lysophosphatidylcholines), Emulsy MU, linoleic acid and sodium taurocholate were weighed at a ratio of 16 : 32 : 52 : 60. Separately, the phosphatide (6), Emulsy MU, capric acid and sodium taurocholate were weighed at a ratio of 20 : 40 : 40. These compositions were melted together with

EP 0 375 785 B1

200 parts of hexane and 200 parts of an alcohol, respectively, and then each solvent was distilled off under reduced pressure. The former composition thus obtained will be referred to as a composition 6-1 while the latter one as a composition 6-2 hereinbelow.

Each composition was dissolved in water to thereby give an aqueous solution having a solid concentration, except for the sodium taurocholate, of 0.2%. The surface activities and spreadabilities on foliage of these aqueous solutions were examined by the same methods as those described in Example 5.

Table 3 shows the results.

Table 3

| Ex. No. | Wetting time at 37°C (sec) | Contact angle on (degrees) | | Spreadability on foliage (score) |
|---|---|---|---|---|
| | | Bees wax | Japan wax | |
| 6-1 | 35.4 | 39° | 39° | 3 |
| 6-2 | 28.9 | 42° | 40° | 4 |

Example 7

Solutions of the following compositions were obtained by using the phosphatides (1) to (4) prepared in Referential Example 1 by the same method as the one described in Example 5.

Each phosphatides, Emulsy MU, linoleic acid and sodium taurocholate were mixed together at a ratio by weight of 17 : 33 : 50 : 67. Then water was added to the resulting mixture to thereby give aqueous solutions of solid concentrations, except for the sodium taurocholate, of 0.5% and 0.2%. The surface activities of these solutions were examined in the same manner as the one described in Example 5.

18

Table 4 shows the results.

Table 4

| Ex. No. | Composition (part) | | | | | Concentration (%) | Result | | | |
| | Phosphatide | | Emulsy MU | Linoleic acid | Sodium taurocholate | | Surface tension at 35°C (dynes/cm) | Wetting time at 37°C (sec) | Contact angle at 30°C on (degrees) | |
| | Kind | Amount | | | | | | | Bees wax | Japan wax |
| Ex. 7-1 | ① | 17 | 33 | 50 | 67 | 0.5 0.2 | 28.9 | 12.5 28.9 | 40 41 | 35 41 |
| Ex. 7-2 | ② | 17 | 33 | 50 | 67 | 0.5 0.2 | 28.5 | 12.3 27.0 | 37 39 | 32 39 |
| Ex. 7-3 | ③ | 17 | 33 | 50 | 67 | 0.5 0.2 | 28.3 | 12.0 26.9 | 35 38 | 31 38 |
| Ex. 7-4 | ④ | 17 | 33 | 50 | 67 | 0.5 0.2 | 28.3 | 11.3 23.6 | 34 36 | 29 35 |

Example 8

The phosphatide (4), Emulsy MUL, linoleic acid and sodium taurocholate were employed at the ratios shown in the following Table 5 and aqueous solutions each having a solid concentration, except for the sodium taurocholate, of 0.5% were obtained in the same manner as the one described in Example 7. The surface activities and spreadabilities onto foliage of these aqueous solutions were measured by the same methods as those described in Example 5.

Table 5 shows the results.

As apparent from Table 5, the solubility was lowered and the surface activities were rather deteriorated when the fatty acids were employed in an amount twice or more as much as the monoglycerides.

Table 5

| Ex. No. / C. Ex. No. | Composition (part) | | | | Result | | | | |
|---|---|---|---|---|---|---|---|---|---|
| | Phosphatide (4) | Emulsy MU | Linoleic acid | Sodium taurocholate | Surface tension at 35°C (dynes/cm) | Wetting time at 37°C (sec) | Contact angle at 30°C on (degrees) Bees wax | Japan wax | Spreadability on foliage (score) |
| C. Ex. 8-1 | 50 | 50 | - | | 29.2 | 40.1 | 48 | 47 | 2 |
| C. Ex. 8-2 | 46 | 46 | 8 | 40 | 28.9 | 31.9 | 45 | 44 | 2 |
| Ex. 8-1 | 44 | 44 | 12 | 40 | 28.8 | 26.2 | 40 | 40 | 3 |
| Ex. 8-2 | 40 | 40 | 20 | 40 | 28.6 | 22.4 | 37 | 35 | 4 |
| Ex. 8-3 | 33 | 33 | 34 | 40 | 28.2 | 19.2 | 36 | 36 | 4 |
| Ex. 8-4 | 29 | 29 | 42 | 42 | 28.4 | 16.3 | 35 | 38 | 4 |
| Ex. 8-5 | 25 | 25 | 50 | 50 | 28.5 | 15.2 | 37 | 38 | 4 |
| Ex. 8-6 | 22 | 22 | 56 | 56 | 28.6 | 16.0 | 40 | 39 | 4 |
| C. Ex. 8-3 | 33 | 67 | - | | 29.6 | 34.6 | 46 | 45 | 2 |
| Ex. 8-7 | 25 | 50 | 25 | 50 | 28.6 | 21.3 | 38 | 39 | 3 |
| Ex. 8-8 | 20 | 40 | 40 | 60 | 28.8 | 14.3 | 38 | 39 | 4 |
| Ex. 8-9 | 17 | 33 | 50 | 60 | 28.8 | 13.7 | 39 | 37 | 4 |
| Ex. 8-10 | 14 | 29 | 57 | 60 | 28.7 | 13.8 | 40 | 34 | 4 |
| Ex. 8-11 | 12.5 | 25 | 62.5 | 65 | 29.0 | 15.2 | 42 | 36 | 3 |

Example 9

The phosphatide (4), Emulsy MTT, oleic acid and sodium taurocholate were weighed at the ratios shown in Table 6. To each mixture was added 300 parts of hexane. The obtained mixture was melted by heating and then the hexane was removed under reduced pressure to thereby give a composition. Each composition thus obtained was dissolved in water and thus an aqueous solution having a solid concentration, except for the sodium taurocholate, of 0.5% was obtained. The surface activities of these aqueous solutions were measured by the same method as the one described in Example 8.

Table 6 shows the results.

Example 10

The phosphatide (4), Emulsy OL, oleic acid and sodium taurocholate were employed at the ratios shown in Table 7. The procedure of Example 9 was repeated to thereby give aqueous solutions each having a solid concentration, except for the sodium taurocholate, of 0.5%. The surface acitivities of these aqueous solutions were measured in the same manner as the one described in Example 9.

Table 7 shows the results.

Table 6

| Ex. No. | Composition (part) | | | | Result | | | |
| | Phos-phatide (A) | Emulsy MTT | Oleic acid | Sodium taurocholate | Surface tension at 35°C (dynes/cm) | Wetting time at 37°C (sec) | Contact angle at 30°C on (degrees) Bees wax | Japan wax |
|---|---|---|---|---|---|---|---|---|
| Ex. 9-1 | 33 | 33 | 34 | 33 | 27.6 | 36.7 | 40.2 | 37.2 |
| Ex. 9-2 | 20 | 40 | 40 | 40 | 28.1 | 32.3 | 42.3 | 39.3 |

Table 7

| Ex. No. | Composition (part) | | | | Result | | | |
| | Phos-phatide (A) | Emulsy OL | Oleic acid | Sodium taurocholate | Surface tension at 35°C (dynes/cm) | Wetting time at 37°C (sec) | Contact angle at 30°C on (degrees) Bees wax | Japan wax |
|---|---|---|---|---|---|---|---|---|
| Ex. 10-1 | 33 | 33 | 34 | 33 | 27.4 | 29.4 | 39.0 | 33.5 |
| Ex. 10-2 | 20 | 40 | 40 | 40 | 27.8 | 24.0 | 41.4 | 34.0 |

Example 11 and Test Example 1

The phosphatide (5) was used in Example 11, while the phosphatide (7) was used in Test Example 1.

In each case, the phosphatide, fatty acid monoglycerides, fatty acids and sodium taurocholate were employed at the ratio shown in Table 8 and an aqueous solution having a solid concentration, except for the sodium taurocholate, of 0.2% was obtained in the same manner as the one described in Example 5. The surface activities of these aqueous solutions were determined by the abovementioned method. Table 8 shows the results. The compositions (aqueous solutions) obtained in Example 11 and Test Example 1 each had a molar ratio of phosphatides : fatty acid monoglycerides : fatty acids of approximately 1 : 3 : 6.

In Test Example 1, the surface activities of compositions which seemed the most effective among those disclosed in Japanese Patent Laid-Open No. 502891/1987 were examined. In these cases, however, no micellar solution was formed but fatty acid crystals were observed at 37°C, except those wherein linoleic and oleic acids were employed, in spite of the addition of the sodium taurocholate. Further, even those wherein linoleic and oleic acids were employed were inferior to the compositions of the present invention in surface activities.

The compositions of Comparative Examples 11-1 to 11-4 were excluded from the scope of the present invention. Namely, fatty acids having eight or less carbon atoms or saturated fatty acids having 14 to 18 carbon atoms were exclusively employed in these cases. In Comparative Example 11-6, saturated fatty acid monoglycerides were exclusively employed as the fatty acid monoglycerides and linoleic acid was employed as the fatty acid. In Comparative Example 11-5, a composition containing phosphatides and fatty acid monoglycerides but no fatty acid was employed. As apparent from Table 8, each composition of Comparative Examples 11-1 to 11-6 were poor in surface activities.

Table 8

| Ex. No.<br>C. Ex. No.<br>T. Ex. No. | Composition (part) | | | | Result | | | | |
|---|---|---|---|---|---|---|---|---|---|
| | Phosphatide | | Fatty acid mono-glyceride Emulsy MU | Fatty acid<br>amount<br>(part) | Sodium tauro cholate | Surface tension at 35°C<br>(dynes/cm) | Wetting time<br>(sec) | | Contact angle at 30°C on (degrees) | |
| | Kind | Amount | | | | | 37°C | 25°C | Bees wax | Japan wax |
| Ex. 11-1 | ⑤ | 16 | 32 | linoleic acid 52 | 60 | 28.3 | 23.3 | 40.3 | 37 | 41 |
| Ex. 11-2 | ⑤ | 16 | 32 | oleic acid 52 | 60 | 27.4 | 32.9 | 48.7 | 38 | 37 |
| C. Ex. 11-1 | ⑤ | 16 | 32 | stearic acid 52 | 100 | ·* | 115* | — | 52* | 54* |
| C. Ex. 11-2 | ⑤ | 17 | 34 | palmitic acid 49 | 100 | ·* | 67.0* | — | 49* | 51* |
| C. Ex. 11-3 | ⑤ | 18 | 36 | myristic acid 46 | 60 | 25.5** | 30.9** | 66.4** | 48** | 48** |
| Ex. 11-3 | ⑤ | 19 | 38 | lauric acid 43 | 40 | 27.1 | 17.3 | 28.8 | 33 | 34 |
| Ex. 11-4 | ⑤ | 20 | 40 | capric acid 40 | 35 | 27.9 | 24.5 | 32.6 | 36 | 43 |
| C. Ex. 11-4 | ⑤ | 21 | 42 | caprylic acid 37 | 20 | 29.2 | 57.2 | 106 | 42 | 45 |

EP 0 375 785 B1

Table 8 (continue)

EP 0 375 785 B1

| Ex. No. C. Ex. No. T. Ex. No. | Composition (part) | | | | | Result | | | | |
|---|---|---|---|---|---|---|---|---|---|---|
| | Phosphatide | | Fatty acid mono-glyceride Emulsy MU | Fatty acid amount (part) | Sodium tauro cholate | Surface tension at 35℃ (dynes/cm) | Wetting time (sec) | | Contact angle at 30℃ on (degrees) | |
| | Kind | Amount | | | | | 37℃ | 25℃ | Bees wax | Japan wax |
| C. Ex. 11-5 | ⑤ | 3 3 | 6 7 | | | 2 8. 4 | 6 7. 4 | 1 6 4 | 4 7 | 4 3 |
| C. Ex. 11-6 | ⑤ | 1 6 | Emulsy MS 3 2 *** | linoleic acid 5 2 | 6 0 | 2 9. 2 | 6 2. 5 | — | 5 2 | 6 5 |
| T. Ex. 1-1 | ⑦ | 1 6 | 3 2 | linoleic acid 5 2 | 6 0 | 2 8. 1 | 2 9. 5 | 4 6. 0 | 3 9 | 4 9 |
| T. Ex. 1-2 | ⑦ | 1 6 | 3 2 | oleic acid 5 2 | 6 0 | 2 7. 4 | 4 3. 8 | 6 7. 2 | 3 8 | 4 4 |
| T. Ex. 1-3 | ⑦ | 1 6 | 3 2 | stearic acid 5 2 | 1 0 0 | — * | 1 3 0 * | — | 5 3 * | 6 5 * |
| T. Ex. 1-4 | ⑦ | 1 7 | 3 4 | palmitic acid 4 9 | 1 0 0 | — * | 7 2. 7 * | — | 4 8 * | 5 9 * |
| T. Ex. 1-5 | ⑦ | 1 8 | 3 6 | myristic acid 4 6 | 6 0 | 2 5. 4 ** | 3 6. 1 ** | 8 4. 4 ** | 4 7 ** | 4 5 ** |

*:   All of the fatty acids could not be dissolved in Comparative Examples 11-1 and 11-2 and Test Examples 1-3 and 1-4. in spite of the addition of sodiuxm taurocholate. Namely crystals were dispersed in the solutions at 40$^\circ$C. Thus apparent values are shown in these cases.

**:   An extremely small amount of fatty acid crystals were observed in Comparative Example 11-3 and Test Example 1-5. Thus accurate data could not be obtained in these cases.

***:   Saturated fatty acid monoglycerides were employed in Comparative Example 11-6.

Example 12

The phosphatide (2), Emulsy MU, linoleic acid and bile acid were employed at the ratios shown in Table 9. Thus aqueous solutions each having a solid concentration, except for the bile acid, of 0.2% were obtained in the same manner as the one described in Example 5. The surface activities of these aqueous solutions were examined in the same manner as the one described in Example 9.

Table 9 shows the results.

Table 9

| Ex. No. | Composition (part) | | | | Result | | | |
|---|---|---|---|---|---|---|---|---|
| | Phos-phatide (2) | Emulsy MU | Linoleic acid | Bile acid | Surface tension at 35℃ (dynes/cm) | Wetting time at 37℃ (sec) | Contact angle at 30℃ on (degrees) Bees wax | Japan wax |
| Ex. 12-1 | 17 | 33 | 50 | sodium taurocholate 8 | 29.0 | 28.3 | 41 | 39 |
| Ex. 12-2 | 17 | 33 | 50 | sodium glycocholate 17 | 31.9 | 42.3 | 43 | 48 |
| Ex. 12-3 | 17 | 33 | 5.0 | sodium glycocholate 25 | 31.2 | 28.8 | 40 | 43 |
| Ex. 12-4 | 17 | 33* | 50 | sodium cholate 25 | 34.3 | 45.8 | 44 | 48 |
| Ex. 12-5 | 17 | 33 | 50 | sodium cholate 25 | 32.1 | 26.9 | 41 | 48 |

28

Example 13

According to the ratios shown in Table 10, the procedure of Example 12 was repeated except that the phosphatide (2) was replaced with the phosphatide (4). Thus aqueous solutions each having a solid concentration, except for the bile acid, of 0.2% were obtained. The surface activities of these aqueous solutions were examined in the same manner as the one described in Example 12.

Table 10 shows the results.

Table 10

| | Composition (part) | | | | Result | | Contact angle at 30 °C on (degrees) | |
|---|---|---|---|---|---|---|---|---|
| Ex. No. | Phos-phatide (4) | Emulsy MU | Linoleic acid | Bile acid | Surface tension at 35°C (dynes/cm) | Wetting time at 37°C (sec) | Bees wax | Japan wax |
| Ex. 13-1 | 17 | 33 | 50 | sodium taurocholate 8 | 28.6 | 29.5 | 42 | 38 |
| Ex. 13-2 | 17 | 33 | 50 | glycocholic acid 8 | 29.0 | 35.7 | 41 | 42 |
| Ex. 13-3 | 17 | 33 | 50 | sodium glycocholate 8 | 30.8 | 31.8 | 41 | 42 |
| Ex. 13-4 | 17 | 33 | 50 | cholic acid 2.5 | 32.2 | 36.6 | 43 | 45 |
| Ex. 13-5 | 17 | 33 | 50 | sodium cholate 8 | 30.6 | 30.4 | 40 | 42 |

Application Examples of the compositions of the present invention:

The effects of accelerating the absorption of lipids in intenstines, accelerating the subcutaneous and transmucosal absorptions of substances, dispersing an inorganic pigment and accelerating the dispersion of cocoa of some compositions obtained in the above Examples were evaluated. The results were as follows.

Application Example 1

Acceleration of the absorption of lipids in intenstine:

This test was conducted in the following manner with rats.

Male Wistar rats weighing approximately 200 g were subjectged to abdominal operation under etherization and the bile ducts of these animals here united.

These rats were fed with a feed (MF powder; mfd. by Oriental Yeast Co., Ltd.) comprising 7.3% of water, 25.1% of crude protein, 5.3% of crude fat, 6.0% of crude ash, 2.3% of fiber, 54.0% of soluble nitrogen-free matters and 880 IU/100 g of vitamin A. Six days after the operation, those showing good postoperative recovery were divided into groups each consisting of six animals.

The composition of the present invention was added to the FM powder and the moisture content was adjusted to 7%. 24 g/day of the feed thus obtained was given to each animal and the feed intake was calculated by substracting the residual feed therefrom.

The difference between the amount of lipids (determined as crude fat in a conventional manner) taken from the fourth to the eighth days after the initiation of the administration, i.e., five days and the amount of lipids in the feces was referred to as the amount of the absorbed lipids. The ratio of the absorbed lipids to the lipids in the given feed was referred to as the absorption ratio. The small amount of lipids excreted from the intestine was ignored.

The absorption ratio of vitamin A was determined by high performance liquid chromatography and calculated in the same manner as the one employed for lipids.

The compositions of Examples 2 and 7-3 were employed. Each composition was melted by heating together with three times as much hexane. Then the hexane was removed under reduced pressure to thereby obtain a composition.

20 parts, in terms of the portion except for the taurocholic acid, of each composition was dissolved in water. Separately, an oil-in-water type emulsion comprising 49 parts of soybean salad oil, one part of an emulsifier (Tween 61; mfd. by Kao Corp.), 0.02 part of vitamin A acetate (high-grade, 1,000,000 IU/g) and 50 parts of water was prepared. 60 parts of this emulsion was mixed with the composition solution and added to 160 parts of the MF powder feed. The resulting mixture was dried to thereby give a moisture content of 7%.

For control, six parts of sodium taurocholate was added to 100 parts of the abovementioned soybean salad oil emulsion and the obtained mixture was added to the MF feed.

The rats were fed with these feeds. Table 11 shows the results.

Table 11

| Composition | Lipid absorption ratio (%) | Vitamin A absortiion ratio (%) |
|---|---|---|
| Ex. 2 | 96 | 90 |
| Ex. 7-3 | 94 | 90 |
| Control | 66 | 52 |

Application Example 2

Acceleration of the subcutaneous and transmucosal absorption of substances:

An external drug comprising indomethacin and a suppository comprising diclofenac sodium were employed.

Test on external drug:

The external drug was applied onto the depilated abdominal skin (10 x 15 cm) of a normal male rabbit in such a manner as to administer 20 mg of indomethacin. The blood indomethacin level was determined 1, 3, 6, 10 and 24 hours after the application by collecting the blood of the animal from the auricular vein and examining the blood by high performance liquid chromatography. The maximum blood indomethacin level at each point was shown.

The compositions of Examples 2 and 7-3 were employed. In each case, the phosphatide, sodium taurocholate, fatty acid monoglycerideds and fatty acids were dissolved in water in this order by heating. After concentration under reduced pressure, an aqueous paste of a solid concentration of 40% was obtained.

20 parts of this composition, three parts of indomethacin, 27 parts of ethanol and 50 parts of water were mixed together to thereby give an external drug. Separately, an external drug comprising two parts of indomethacin, 30 parts of dimethyl sulfoxide, 98 parts of ethanol and 70 parts of water was used as a control.

As the result of the subcutaneous absorption test on rabbits, the control drug showed the maximum blood indomethacin level of 180 ng/ml, while those comprising the compositions of Examples 2 and 7-3 showed the maximum blood indomethacin levels of 1530 and 1740 ng/ml, respectively.

Test on suppository:

A suppository comprising 15 mg of diclofenac sodium was rectally administered to a normal male rabbit. 10, 30 and 60 minutes after the administration, the blood of the animal was collected from its auricular vein. The plasma was separated and extracted with benzene. Then the diclofenac sodium in the extract was determined by gas chromatography with the use of an electron capture detector.

Similar to the above Application Example 1, the compositions of Example 2 and 10-2 were employed. Eight parts of each composition was dissolved in 25 parts of a mixture of ethanol with hexane (1 : 2) and five parts of diclofenac sodium and 87 parts of cacao butter were added thereto under heating. After removing the solvent under reduced pressure, the residue was formulated into cylindrical suppositories each weighing 0.3 g. Separately, five parts of diclofenac sodium was mixed with 95 parts of cacao fat and the mixture was formulated into suppositories, similar to the abovementioned ones, as a control.

Table 12 shows the results of the administration of these suppositories to rabbits.

Table 12

| Composition | Blood diclofenac sodium level (ng/ml) | | |
|---|---|---|---|
| | after 10 min | after 30 min | after 60 min |
| Ex. 2 | 20.3 | 13.9 | 4.2 |
| Ex. 10-2 | 17.2 | 11.4 | 3.5 |
| Control | 8.9 | 4.7 | 1.8 |

Application Example 3

Dispersion of inorganic pigment (titanium white):

A 0.25% (w/vol) aqueous solution of each composition was prepared. 20 ml of this aqueous solution and 1 g of titanium white (JR-701; mfd. by Teikoku Kako Kogyo K.K.) were introduced into a Nessler's tube and Vigorously shaken vertically to thereby disperse the pigment in the solution. The obtained dispersion was allowed to stand in a room for one and three dads and the sedimentation of the pigment particles was observed. Table 13 shows the results.

Table 13

| Composition | Sedimentation after 1 day | Sedimentation after 3 days |
|---|---|---|
| Ex. 7-3 | 0 | 1.8 |
| Ex. 7-4 | 0 | 1.7 |
| Ex. 8-3 | 0 | 1.9 |
| Ex. 8-9 | 0 | 1.7 |
| Ex. 11-3 | 0 | 1.2 |
| Ex. 11-4 | 0 | 1.3 |
| Control (water) | 3.1 | 3.0 |

In the case of water, almost all particles were sedimented after one hour. In the case of each composition of the present invention, on the other hand, a large amount of the pigment powder remained in the upper part of the solution even after two days, though some particles were sedimented.

Application Example 4

Wetting of cocoa powder:

An aqueous solution of each composition was prepared in such a manner as to give a solid concentration, except for the bile acids etc., of 0.2%. 100 ml portions of this solution were poured into beakers. 1 g of a cocoa powder (Hersey Cocoa; mfd. by Fujiya Confectionery Co., Ltd.) was placed on each solution at 20°C while stirring with a magnetic stirrer at such a constant rate as to give a slight depression on the surface of the solution. Then the time required for substantially dispersing the cocoa in the solution was measured. Each composition was tested thrice and the mean was determined. Table 14 shows the results.

Table 14

| Composition | Result |
|---|---|
| Control (water at 20°C) | 2 min 58 sec |
| Ex. 7-3 | 1 min 16 sec |
| Ex. 7-4 | 1 min 8 sec |
| Ex. 8-3 | 1 min 38 sec |
| Ex. 11-3 | 1 min 3 sec |

Application Example 5

Acceleration of the dispersion of cocoa:

The compositions of Examples 6-1 and 7-4 were employed. 3 g. in terms of solid content, of each composition was dissolved in 40 g of ethanol. The obtained solution was homogenized together with 147 g of a cocoa powder (Hersey Cocoa; mfd. by Fujiya Confectionery Co., Ltd.) by vigorously stirring in a Kenwood mixer. Then the homogenized mixture was dried by removing the alcohol under reduced pressure. The residue was milled in a mortar. Thus instant cocoa products (1) and (2) were obtained.

Separately, an instant cocoa product (3) was prepared from Ryoto Ester S-1670-S in the same manner.

2 g of each product was gently placed on the surface of 50 ml of water at 20°C in a 50-ml beaker. Then the time required for substantially sedimenting the whole of the cocoa into water was measured. Each

EP 0 375 785 B1

sample was examined four times and the mean was determined. The results were as follows. Namely, the instant cocoa products comprising the compositions of the present invention were highly dispersible when stirred in cold water.

| instant cocoa product (1): | 2 min 17 sec; |
| instant cocoa product (2): | 2 min 9 sec; and |
| instant cocoa product (3): | 7 min 2 sec |

Next, Examples of the lipid composition of the present invention satisfying the abovementioned requirement 2 will be given.

Example 14

Commercially available soybean phospholipids were defatted with acetone and fractionated with an aqueous alcohol to thereby give a phosphatide containing 70% of phosphatidylcholine. The product was treated with phospholipase A-2 (Lecithane 10-L; mfd. by Novo) and treated with acetone to thereby remove fatty acids. Then it was further treated with an aqueous alcohol and subjected to silica gel chromatography with the use of an aqueous alcohol. Thus a phosphatide containing 97% of lyso phosphatidylcholine was obtained. The obtained phosphatide was employed as the lyso phosphatidylcholine.

As the unsaturated fatty acid monoglycerides, monoglycerides mainly comprising linoleic acid, namely, Emulsy MU (mfd. by Riken Vitamin Co., Ltd.) comprising 74% of linoleic acid, 13% of oleic acid, 8% of palmitic acid and 2.6% of stearic acid and having a monoester content of 95% and an iodine number of 116; and Emulsy MO (mfd. by Riken Vitamin Co., Ltd.) comprising 47% of linoleic acid, 14% of oleic acid and 37% of plamitic acid and having a monoester content of 93% and an iodine number of 72; and monoglycerides mainly comprising oleic acid, namely Emulsy OL (mfd. by Riken Vitamin Co., Ltd.) comprising 74% of oleic acid, 5% of linoleic aicd, 5% of palmitoleic acid and 4% of palmitic acid and having a monoester content of 93% and an iodine number of 67 were employed.

As the fatty acids, lauric acid (NAA-122; mfd. by Nippon Oils and Fats Co., Ltd., purity of 99%) and capric acid (Lunac 10-95; mfd. by Kao Corp., purity of 95%) were employed.

As the bile acid derivative, sodium taurocholate (mfd. by Difco Lab., purity of 70%) was employed.

For comparison, myristic acid (NAA 142; mfd. by Nippon Oils and Fats Co., Ltd., purity of 99%) and caprylic acid (Lunac 8-95; mfd. by Kao Corp., purity of 95%) which were excluded from the scope of the present invention were employed.

Each composition listed in Table 15 was melted together with a mixture of hexane with an alcohol (1 : 1) three times by weight as much as the same under heating. After removing the solvent under reduced pressure, a composition was obtained.

Each composition was formulated into an aqueous solution having a solid concentration, except for the sodium taurocholate, of 0.2% and the surface activities of the obtained solution were determined by the following methods.

(1) Effect of lowering surface tension:

The surface tension of each sample was measured at 35°C with a surface tension meter (CBVP A-3; mfd. by Kyowa Kagaku K.K.) provided with a platinum plate.

(2) Canvas disc wetting time (time needed for sedimentation thereof):

The permeability of each sample was determined at 37°C and 25°C by using a thick canvas cloth of 1 inch in diameter according to the procedure described in "Kaimen Kasseizai Binran" (p. 860, Sangyo Tosho K.K. (1960)).

(3) Contact angle:

The angles of contact with bees wax and Japan wax of each sample were determined at 30°C with a contact angle meter (Elmer-13; mfd. by Elmer Kogaku K.K.).

Furthermore, the spreadability of each sample onto foliage was evaluated in the abovementioned manner.

34

Table 15 summarizes the results.

Table 15

| Ex. No. C. Ex. No. | Composition (part) | | | | Result | | | | | |
|---|---|---|---|---|---|---|---|---|---|---|
| | Lyso-phosphatidyl-choline | Mono-glyceride | Fatty acid | Sodium tauro-cholate | Surface tension 0.2%, at 35℃ (dynes/cm) | Wetting time 0.2% (sec) | | Contact angle, 0.2%, at 30 ℃ on (degrees) | | Spread-ability on foliage (score) |
| | | | | | | 37℃ | 25℃ | Bees wax | Japan wax | |
| C. Ex. 12-1 | 100 | — | — | | 37.8 | 201 | 603 | 64 | 67 | |
| C. Ex. 12-2 | 33 | Emulsy MU 67 | — | | 29.2 | 74.2 | 205 | 44 | 50 | |
| C. Ex. 12-3 | 18 | Emulsy MU 36 | myristic acid 46 | 60 | 25.4* | 36.1* | 8 1.4* | 47* | 45* | 3* |
| Ex. 14-1 | 19 | Emulsy MU 38 | lauric acid 43 | 40 | 25.9 | 22.6 | 41.5 | 34 | 39 | 4 |
| Ex. 14-2 | 20 | Emulsy MU 40 | capric acid 40 | 35 | 27.2 | 26.4 | 32.8 | 35 | 42 | 3 |
| C. Ex. 12-4 | 21 | Emulsy MU 42 | caprylic acid 37 | 20 | 27.8 | 51.5 | 114 | 39 | 51 | 2 |
| Ex. 14-3 | 20 | Emulsy MO 40 | capric acid 40 | 35 | 27.4 | 23.9 | — | 39 | 42 | 3 |
| Ex. 14-4 | 14 | Emulsy MU 43 | capric acid 43 | 35 | 27.3 | 18.7 | — | 35 | 43 | 4 |
| Ex. 14-5 | 40 | Emulsy MU 40 | lauric acid 20 | — | 28.6 | 38.0 | — | 38 | 45 | 3 |
| Ex. 14-6 | 19 | Emulsy OL 38 | lauric acid 43 | 20 | | 29.4 | — | 35 | 43 | 3 |

*: In Comparative Example 12-3, a small amount of fatty acids crystallized out at 37℃. Thus the data regarding the surface activities of this example are not accurate.

Example 15

Commercially available soybean phospholipids were defatted with acetone and fractionated with an aqueous alcohol to thereby give phosphatides containing 70% of phosphatidylcholine. Then the obtained product was treated with phospholipase A-2 (Lecithase 10-L; mfd. by Novo) and treated with acetone to thereby remove fatty acids. Next, it was treated with an aqueous alcohol and subjected to silica gel chromatography with the use of an aqueous alcohol. Thus phosphatides containing 87% of lyso phosphatidylcholine were obtained.

17 parts, in terms of pure compound, of the lysophosphatidylcholine thus obtained, 33 parts of Emulsy MU, 50 parts of lauric acid and 20 parts of crystalline glycocholic acid (mfd. by Sigma) were formulated into a composition in the same manner as the one described in Example 14. The composition was then formulated into aqueous solutions of solid concentrations, except for the glycocholic acid, of 0.2 and 0.5% and the surface activities of these aqueous solutions were examined by the same methods as those described in Example 14. The results were as follows.

0.2% aqueous solution:

    surface tension: 26.7 dynes/cm,
    canvas disk wetting time at 37°C: 27.3 sec,
    contact angle on bees wax: 35°, and
    contact angle on Japan wax: 41°.

0.5% aqueous solution:

    canvas disk wetting time at 37°C: 6.7sec.

Example 16

An aqueous paste of yolk phospholipids (Purified Yolk Lecithin; mfd. by Asahi Chemical Industry Co., Ltd.) were treated in the same manner as the one descrived in Example 14 to thereby give a phosphatide containing 98% of lysophosphatidylcholine.

17 parts of the obtained phosphatide, 33 parts of Emulsy MU, 50 parts of capric acid and 30 parts of sodium taurocholate were blended together and thus a composition was obtained in the same manner as the one described in Example 14. The surface activities of this composition were examined similar to Example 15. The results were as follows.
    Surface tension: 27.2 dynes/cm,
    canvas disk wetting time at 37°C: 27.1 sec,
    contact angle on bees wax: 38°, and
    contact angle with Japan wax: 41°.

Example 17

The procedure of Example 16 was repeated except that the lysophosphatidylcholine, Emulsy MU, capric acid and sodium taurocholate were employed at a ratio by weight of 20 : 40 : 40 : 40. The surface activities of the obtained composition were examined similar to Example 16. The results were as follows:
    surface tension: 27.8 dynes/cm,
    canvas disk wetting time at 37°: 32.1 sec,
    contact angle with bees wax: 40°, and
    contact angle with Japan wax: 43°.

Application Examples of the compositions of the present invention, requirement 2:

The effects of dispersing an inorganic pigment (titanium white), wetting a cocoa powder and accelerating the dispersion of a cocoa powder of the compositions of the above Examples 14-1 and 14-2 were examined.

Application to Example 6

Dispersion of inorganic pigment (titanium white):

The procedure of Application Example 3 was repeated. Table 16 shows the results.

Table 16

| Composition | Sedimentation after 1 day | Sedimentation after 3 days |
|---|---|---|
| Ex. 14-1 | 0 | 1.2 |
| Ex. 14-2 | 0 | 1.3 |
| Control (water) | 3.1 | 3.0 |

In the case of water alone, almost all particles were sedimented after one hour. In the cases of each composition of the present invention, on the other hand, a large amount of the pigment powder remained in the upper part of the solution even after two days, though some particles were sedimented.

Application Example 7

Wetting of cocoa powder:

The procedure of Application Example 4 was repeated. Each composition was tested thrice and the mean was determined. Table 17 shows the results. As apparent from Table 17, each composition of the present invention accelerated the wetting of the cocoa powder.

Table 17

| Composition | Result |
|---|---|
| Control (water at 20°C) | 2 min 57 sec |
| Ex. 14-1 | 1 min 9 sec |
| Ex. 14-2 | 1 min 15 sec |

Application Example 8

Acceleration of the dispersion of cocoa:

The composition of Example 14-1 was treated in the same manner as the one described in Application Example 5 to thereby give an instant cocoa product (1).

Separately, Ryoto Ester S-1670-S was treated likewise to thereby give another instant cocoa product (2).

2 g of each product was gently placed on the surface of 50 ml of water at 20°C in a 50-ml beaker. Then the time required for substantially sedimenting the whole of the cocoa into water was measured. Each sample was tested four times and the mean was determined. The results were as follows. Further, the instant cocoa product comprising the composition of the present invention was highly dispersible when stirred in cold water.

| instant cocoa product (1): | 2 min 20 sec, and |
|---|---|
| instant cocoa product (2): | 7 min 2 sec |

Next, the lipid composition of the present invention satisfying the abovementioned requirement 3 will be described.

Example 18

29 parts of the phosphatide (3), 29 parts of Poem M-300 and 42 parts of lauric acid were dissolved in 200 parts of hexane. After distilling off the solvent under reduced pressure, a composition was obtained. This composition was dissolved in water to thereby give a 0.8% solution. The surface activities of this solution and the spreadability onto foliage of a 0.1% solution were examined each by the abovementioned method.

As a result, the sample showed a permeation period at 37°C in the canvas disc method of 1.7 sec, a surface tension at 35°C of 25.2 dynes/cm and an contact angle on bees wax of 33°. The spreadability onto foliage of the sample was evaluated to be 5.

Example 19

A composition comprising 15 parts of the phosphatide (3), 35 parts of Poem M-300, 50 parts of lauric acid and 10 parts of sodium taurocholate was prepared in the same manner as the one described in Example 18. The obtained solution was formulated into an aqueous solution having a concentration, except for the sodium taurocholate, of 0.8%. The surface activities of this aqueous solution were examined similar to Example 18.

As a result, the sample showed a canvas disk wetting time of 1.0 sec, a surface tension of 25.2 dynes/cm and a contact angle with bees wax of 31°. The spreadability of the same onto foliage was evaluated to be 5.

Example 20

37 parts of the phosphatide (3), 37 parts of Poem M-300 and 26 parts of lauric acid were dispersed in 1500 parts of water by heating. The obtained dispersion was treated with a high-pressure homogenizer (3205-200 LH; mfd. by Sanwa Kikai K.K.) at 50°C under 200 kg/cm$^2$ to thereby give a micellar solution. This solution was concentrated to give a volume of 500 parts. Thus a composition in the form of an aqueous paste was obtained. This pasty composition was dissolved in water to thereby give a 0.8% aqueous solution. The surface activities of this solution were examined similar to Example 18.

As a result, the sample showed a wetting time of 3.2 sec, a surface tensiodn of 25.4 dynes/cm and a contact angle on bees wax of 35°.

Example 21 to 24

According to each composition listed in Table 18, phosphatides, fatty acid monoglycerides, fatty acids and sodium taurocholate were weighed. To the obtained mixture was added water. The resulting mixture was heated to 50°C and dissolved and/or dispersed with an ultrasonic vibrator (UD-200; mfd. by Tomy Seiko K.K.). Thus 300-g portions of aqueous solutions having concentrations, except for the sodium taurocholate, of 1, 0.73 and 0.2% were prepared. The surface activities of each aqueous solution thus obtained were examined in the same manner as the one described in Example 18.

Table 18 summarizes the results.

Table 18

| Ex. No. | Composition (part) | | | | Concn. (%) | Result | | | |
|---|---|---|---|---|---|---|---|---|---|
| | Phos-phatide * | Mono-glyceride * | Fatty acid * | Sodium tauro-cholate | | Surface tension (dynes/cm) | Canvas disk Wetting time (sec) | Contact angle on bees wax (degrees) | Spread-ability on foliage (score) |
| 21 | (3) 2 0 | Poem M-100 4 8 | caprylic acid 3 2 | none none | 0. 7 3 | 2 4. 8 | 1. 9 | 3 3 | 5 |
| 22 | (4) 2 0 | Poem M-200 3 3 | capric acid 4 7 | none none | 1. 0 0. 2 | 2 3. 9 2 3. 7 | ≒ 0 5. 4 | 2 9 | 5 |
| 23 | (3) 1 5 | Poem M-300 3 5 | lauric acid 5 0 | none 5 | 1. 0 0. 2 | 2 5. 1 | ≒ 0 3. 9 | 3 2 | 5 |
| 24 | (5) 1 4 | Poem C-100 3 4 | coconut oil fatty acid 5 2 | none none | 1. 0 | 2 4. 8 | 3. 4 | 3 4 | 5 |

* : The upper figure shows a component, while the lower one its amount (part).

Example 25

30 parts of the phosphatide (6) (yolk phosphatide), 40 parts of Poem M-200, 40 parts of capric acid, 20 parts of sodium taurocholate, 100 parts of hexane and 100 parts of ethanol were melted together. Then the solvent was removed under reduced pressure to thereby give a composition (Example 25-1).

Separately, a composition comprising 15 parts of the phosphatide (6), 34 parts of Poem M-200, 51 parts of capric acid and 30 parts of taurocholate was prepared likewise (Example 25-2).

The compositions of Examples 25-1 and 25-2 were dissolved in water to thereby give each an aqueous solution having a concentration, except for the sodium taurocholate, of 0.2%. The surface activities of these solutions were examined in the same manner as those employed in Examples 21 to 24.

Table 19 shows the results.

Table 19

| Ex. No. | Surface tensidon (dynes/cm) | Wetting time (sec) | Contact angle (degrees) |
| --- | --- | --- | --- |
| 25-1 | 26.1 dynes/cm | 8.3 | 35° |
| 25-2 | 25.8 dynes/cm | 5.2 | 33° |

Example 26

Compositions comprising phosphatides (1) to (5), Poem M-200, capric acid ($C_{10}$) and sodium taurocholate at a ratio by weight of 17 : 33 : 50 : 67 were prepared. To each composition was added water to thereby give aqueous solutions having concentrations, except for the sodium taurocholate, of 0.5 and 0.2%. The surface activities of these aqueous solutions were examined in the abovementioned manner.

Table 20 shows the results.

EP 0 375 785 B1

Table 20

| Ex. No. | Composition (part) | | | | Concn. (%) | Result | | Contact angle, at 30°C on (degrees) | | Spread-ability on foliage (score) |
|---|---|---|---|---|---|---|---|---|---|---|
| | Phos-phatide | Poem M-200 | Capric acid | Sodium lauro-cholate | | Surface tension at 35°C (dynes/cm) | Canvas disk Wetting time at 37 °C (sec) | Bees wax | Japan wax | |
| 26-1 | (1) 17 | 33 | 50 | 67 | 0.5 0.2 | 25.0 24.8 | ≒0 2.2 | 26 25 | 26 19 | 5 |
| 26-2 | (2) 17 | 33 | 50 | 67 | 0.5 0.2 | 24.9 24.7 | ≒0 2.4 | 24 25 | 25 17 | 5 |
| 26-3 | (3) 17 | 33 | 50 | 67 | 0.5 0.2 | 24.8 24.7 | ≒0 2.4 | 21 21 | 24 16 | 5 |
| 26-4 | (4) 17 | 33 | 50 | 67 | 0.5 0.2 | 24.8 24.7 | ≒0 2.5 | 20 22 | 22 14 | 5 |
| 26-5 | (5) 17 | 33 | 50 | 67 | 0.5 0.2 | 24.7 24.6 | ≒0 2.6 | 19 20 | 22 14 | 5 |

Example 27

The procedure of Example 26 was repeated to thereby give compositions comprising the phosphatides (1) to (4), Poem C-100, coconut oil fatty acid and sodium taurocholate at a ratio by weight of 17 : 33 : 50 : 67. To each composition thus obtained was added water to thereby give aqueous solutions having concentrations, except for the sodium taurocholate, of 0.5 and 0.2%. The surface activities of these aqueous solutions were examined in the abovementioned manner.

Table 21 shows the results.

Table 21

| Ex. No. | Composition (part) | | | | Concn. (%) | Result | | | | |
|---|---|---|---|---|---|---|---|---|---|---|
| | Phosphatide* | Poem C-100 | Coconut oil fatty acid | Sodium taurocholate | | Surface tension at 35°C (dynes/cm) | Canvas disk Wetting time at 37°C (sec) | Contact angle, at 30°C on (degrees) Bees wax | Contact angle, at 30°C on (degrees) Japan wax | Spread-ability on foliage (score) |
| 27-1 | (1) 17 | 33 | 50 | 67 | 0.5 / 0.2 | 26.6 / 25.5 | ≤0 / 12.4 | 35 / 36 | 35 / 34 | 5 |
| 27-2 | (2) 17 | 33 | 50 | 67 | 0.5 / 0.2 | 26.4 / 25.5 | ≤0 / 11.3 | 34 / 34 | 33 / 33 | 5 |
| 27-3 | (3) 17 | 33 | 50 | 67 | 0.5 / 0.2 | 26.3 / 25.3 | ≤0 / 11.0 | 34 / 33 | 33 / 32 | 5 |
| 27-4 | (4) 17 | 33 | 50 | 67 | 0.5 / 0.2 | 26.3 / 25.2 | ≤0 / 10.8 | 32 / 33 | 33 / 32 | 5 |

43

Example 28

17 parts of the phosphatide (7), 33 parts of Poem M-200, 50 parts of capric acids and 33 parts of sodium taurocholate were treated in the same manner as those described in Examples 21 to 24 to thereby give aqueous solutions having concentrations, except for the sodium taurocholate, of 0.2 and 0.5% (Example 28-1). Separately, the phosphatide (7), Poem C-100, coconut oil fatty acid and sodium taurocholate were used at the same ratio as the one specified above and treated likewise to thereby give 0.2 and 0.5% aqueous solutions (Example 28-2). The surface activities of these aqueous solutions were examined in the abovementioned manner.

Table 22 shows the results.

EP 0 375 785 B1

## Table 22

| Ex. No. | Concn. (%) | Surface tension at 35°C (dynes/cm) | Canvas disk wetting time at 37°C (sec) | Contact angle at 30°C on (degrees) | | Spreadability on foliage (score) |
|---|---|---|---|---|---|---|
| | | | | Bees wax | Japan wax | |
| 28-1 | 0.2 | 24.5 | 1.0 | 21 | 11 | 5 |
| | 0.5 | 24.6 | 5.0 | 22 | 9 | 5 |
| 28-2 | 0.2 | 25.3 | 7.1 | 32 | 32 | 5 |
| | 0.5 | 25.5 | 3.9 | 32 | 31 | 5 |

Example 29

The procedure of Example 19 was repeated by using the phosphatide (5), Poem M-200, capric acid and sodium taurocholate to thereby give compositions shown in Table 23. These compositions were formulated into aqueous solutions having concentrations, except for the sodium taurocholate, of 0.5 and 0.2%. The surface activities of these aqueous solutions were examined in the abovementioned manner.

EP 0 375 785 B1

Table 23 shows the results.

## Table 23

| Ex. No. | Composition (part) | | | | Concn. (%) | Result | | | | Note |
| | Phos-phatide (5) | Poem M-200 | Capric acid | Sodium tauro-cholate | | Surface tension at 35℃ (dynes/cm) | Canvas disk wetting time at 37℃ (sec) | Contact angle, on bees wax at 30℃ (degrees) | Spread-ability on foliage (score) | |
|---|---|---|---|---|---|---|---|---|---|---|
| 29-1 | 9 | 3 8 | 5 3 | 5 | 0.5 / 0.2 | 2 3.2 / ... | ≒ 0 / 5.0 | 1 4 / — | 5 | liable to crystal-lize |
| 29-2 | 1 7 | 3 4 | 4 8 | -- | 0.5 / 0.2 | 2 3.4 / .. | ≒ 0 / 5.4 | 1 7 / — | 5 | |
| 29-3 | 2 9 | 2 9 | 4 1 | — | 0.5 / 0.2 | 2 4.1 / — | ≒ 0 / 7.7 | 2 1 / — | 5 | |
| 29-4 | 9 | 3 8 | 5 3 | 2 0 | 0.5 / 0.2 | 2 3.8 / — | ≒ 0 / 2.0 | 1 8 / — | 5 | |
| 29-5 | 1 7 | 3 4 | 4 8 | 1 5 | 0.5 / 0.2 | 2 4.0 / -- | ≒ 0 / 2.7 | 2 0 / — | 5 | |
| 29-6 | 1 1 | 4 4 | 4 4 | 5 | 0.5 / 0.2 | 2 3.5 / — | ≒ 0 / 2.3 | 1 3 / — | 5 | |
| 29-7 | 1 4 | 5 7 | 2 9 | 5 | 0.5 / 0.2 | 2 3.7 / — | ≒ 0 / 2.6 | 1 5 / — | 5 | |

Example 30

The phosphatide (4), Poem M-300 and lauric acid were weighed at the ratios shown in Table 24. To each mixture thus obtained was added twice as much hexane. After melting together by heating, the hexane was removed under reduced pressure to thereby give a composition. The composition was formulated into a 0.2% aqueous solution. The surface activities of these aqueous solutions thus obtained were examined in the abovementioned manner.

Table 24 shows the results.

Table 24

| Ex. No. | Composition (part) | | | Result | | | | |
| | Phosphatide (A) | Poem M-300 | Lauric acid | Surface tension at 35°C (dynes/cm) | Canvas disk wetting time at 37°C (sec) | Contact angle at 30°C on (degrees) Bees wax | Japan wax | Spread-ability on foliage (score) |
|---|---|---|---|---|---|---|---|---|
| 30-1 | 72 | 8 | 20 | 29.2 | 72.5 | 43 | 43 | 3 |
| 30-2 | 57 | 14 | 29 | 27.5 | 39.2 | 36 | 37 | 4 |

49

Example 31

The phosphatide (4), Poem M-200, various fatty acids and sodium taurocholate were weighed at the ratios shown in Table 25. To each mixture thus obtained was added 300 parts of hexane. After melting together by heating, the hexane was removed under reduced pressure. Then the obtained composition was formulated into an aqueous solution having a concentration, except for the sodium taurocholate, of 0.2%. The surface activities of these aqueous solutions thus obtained were examined in the abovementioned manner.

Table 25 shows the results.

EP 0 375 785 B1

T a b l e   2 5

| Ex. No. | Composition (part) | | | | Result | | | | |
|---|---|---|---|---|---|---|---|---|---|
| | Phos-phatide (4) | Poem M-200 | Fatty acid * | Sodium tauro cho-late | Surface tension at 35°C (dynes/cm) | Canvas disk wetting time at 37°C (sec) | Contact angle at 30°C on (degrees) Bees wax | Japan wax | Spread-ability on foliage (score) |
| 31-1 | 18 | 27 | palmitic acid 55 | 55 | 27.8 | 39.0 | 41 | 35 | 3 |
| 31-2 | 20 | 29 | myristic acid 51 | 55 | 27.1 | 24.0 | 36 | 31 | 4 |
| 31-3 | 20 | 40 | lauric acid 40 | 50 | 24.9 | 9.1 | 18 | 15 | 5 |
| 31-4 | 16 | 32 | linoleic acid 52 | 60 | 29.1 | 19.1 | 43 | 38 | 4 |

* : The upper figure shows a component, while the lower one its amount (part).

51

Example 32

A composition comprising 13 parts of the phosphatide (2), 36 parts of Poem M-200 and 51 parts of capric acid was mixed with eight parts of sodium taurocholate. 15 parts of glycocholic acid, 20 parts of sodium glycocholate, 25 parts of cholic acid or 25 parts of sodium cholate to thereby give each a composition in the same manner as the one described in Example 31. Each composition thus obtained was formulated into an aqueous solution having a concentration, except for the bile acid, of 0.2%. The surface activities of these aqueous solutions thus obtained were examined in the abovementioned manner.

Table 26 summarizes the results.

Table 26

| Ex. No. | Composition (part) | | | | Result | | |
|---|---|---|---|---|---|---|---|
| | Phosphatide (2) | Poem M-200 | Capric acid | Bile acid * | Surface tension at 35°C (dynes/cm) | Canvas disk wetting time at 37°C (sec) | Contact angle on bees wax at 30°C (degrees) |
| 32-1 | 13 | 36 | 51 | taurocholic acid 8 | 23.4 | 5.2 | 24 |
| 32-2 | 13 | 36 | 51 | glycocholic acid 15 | 25.5 | 7.8 | 26 |
| 32-3 | 13 | 36 | 51 | sodium glycocholate 20 | 25.0 | 5.4 | 23 |
| 32-4 | 13 | 36 | 51 | cholic acid 25 | 27.7 | 8.5 | 26 |
| 32-5 | 13 | 36 | 51 | sodium cholate 25 | 26.0 | 4.9 | 23 |

* : The upper figure shows a component, while the lower one its amount (part).

Comparative Example 13

Compositions falling within a range which seemed the most effective based on the disclosure of Japanese Patent Laid-Open No. 502891/1987 (c.f. Claim 11) were prepared by the following method. When

$C_{18}$ lipids were selected, the ratio by weight of the phosphatides, monoglycerides and fatty acids was approximately 6 : 35 to 19 : 31 : 50.

With the use of the phosphatide (7) (soybean lysophosphatidylcholine), compositions shown in Table 27 were prepared by the same method as those described in Example 21 to 24. The sruface activities of a 0.2% aqueous solution of each composition were examined likewise.

Table 27 shows the results. As apparent from Table 27, each composition was inferior to that of the present invention. No composition prepared in this Comparative Example could be dissolved and/or dispersed unless sodium taurocholate was added.

Table 27

| C. Ex. No. | Composition (part) | | | | Result | | Contact angle, at 30°C on (degrees) | |
| --- | --- | --- | --- | --- | --- | --- | --- | --- |
| | Phosphatide (7) | Mono-glyceride Emulsy MU | Fatty acid | Sodium taurocholate | Surface tension at 35°C (dynes/cm) | Canvas disk Wetting time at 37°C (sec) | Bees wax | Japan wax |
| 13-1 | 100 | | | | 37.8 | 201 | 64 | 67 |
| 13-2 | 16 | 32 | oleic acid 52 | 60 | 27.4 | 47.8 | 39 | 42 |
| 13-3 | 16 | 32 | stearic acid 52 | 100 | (27.2)* | 130* | 61* | 65* |
| 13-4 | 17 | 34 | palmitic acid 49 | 100 | (25.5)* | 72* | 49* | 58* |
| 13-5 | 16 | Emulsy MS 32 | linoleic acid 52 | 100 | 29.2 | 89 | 56 | 67 |
| 13-6 | 16 | Emulsy MS 32 | stearic acid 52 | 100 | 33.2* | 1015 | 72* | 80* |

* : In Comparative Examples 13-3, 13-4 and 13-6, the fatty acids could not be completely dissolved. Thus these fatty acids crystallized out at 25 to 40 °C. Therefore the surface activities in these cases were apparent ones.

Application Example of the compositions of the present invention, requirement 3:

The effects of accelerating the absorption of lipids in intestines, accelerating the subcutaneous or tarnsmucosal absorptions of substances, dispersing an inorganic pigment and accelerating the dispersion of a cocoa powder of some compositions obtained in the above Examples were examined. The results were as follows.

Application Example 9

Acceleration of the absorption of lipids in intenstine:

By using the compositions of Examples 22 and 26-4, the procedure of Application Example 1 was repeated, except that six parts of sodium taurocholate was added to the composition of Example 22. Separately, 100 parts of a soybean salad oil emulsion containing six parts of sodium taurocholate was added to the MF feed as a control.

Table 28 shows the results.

Table 28

| Composition | Lipid absorption ratio (%) | Vitamin A absorption ratio (%) |
|---|---|---|
| Ex. 22 | 95 | 92 |
| Ex. 26-4 | 97 | 91 |
| Control | 66 | 52 |

Application Example 10

Acceleration of the subcutaneous and transmucosal absorption of substances:

The procedures of Application Example 2, wherein an external drug comprising indomethacin and a suppository comprising diclofenac sodium were employed, was repeated.

In the case of the external drug, the compositions of Examples 22 and 26-4 were treated in the same manner as the one described in Application Example 2. The same control drug as the one used in Application Example 2 was also used as a control.

As the result of the subcutaneous absorption test on rabbits, the maximum blood indomethacin level of the control was 180 mg/ml, while those of the compositions of Examples 22 and 26-4 were 1920 and 2190 mg/ml, respectively.

In the case of the suppository test, the compositions of Examples 30-1 and 31-4 were treated in the same manner as the one described in Application Example 2. The same control drug as the one used in Application Example 2 was also used as a control.

Table 29 shows the results of the administration test on rabbits.

Table 29

| Composition | Blood diclofenac sodium | | level (mg/ml) |
|---|---|---|---|
| | after 10 min | after 30 min | after 60 min |
| Ex. 30-1 | 14.9 | 8.0 | 2.9 |
| Ex. 31-4 | 15.3 | 9.2 | 3.1 |
| Control | 8.9 | 4.7 | 1.8 |

Application Example 11

Dispersion of inorganic pigment (titanium white):

The procedure of Example 3 was repeated. Table 30 shows the results.

Table 30

| Composition | Sedimentation after 1 day | Sedimentation after 3 days |
|---|---|---|
| Ex. 26-4 | 0 | 1.0 |
| Ex. 27-3 | 0 | 2.0 |
| Ex. 27-4 | 0 | 2.0 |
| Ex. 29-3 | 0 | 1.7 |
| Ex. 31-3 | 0 | 1.6 |
| Control (water alone) | 3.1 | 3.0 |

In the case of water, almost all particles were sedimented after one hour. In the case of each composition of the present invention, on the other hand, a large amount of the pigment powder remained in the upper part of the solution even after two days, though some particles were sedimented.

Application Example 12

Wetting of cocoa powder:

The procedure of Application Example 4 was repeated. Each composition was examined thrice and the mean was determined. Table 31 shows the results.

Table 31

| Composition | Result |
|---|---|
| Control (water at 20 °C) | 2 min 57 sec |
| Ex. 27-3 | 1 min 21 sec |
| Ex. 27-4 | 1 min 11 sec |
| Ex. 29-3 | 1 min 15 sec |
| Ex. 29-5 | 1 min 8 sec |

Application Example 13

Acceleration of the dispersion of cocoa:

By using the compositions of Examples 29-5 and 31-3, instant cocoa products (1) and (2) were prepared.

Separately, Ryoto Ester S-1670-S was treated likewise to thereby give another instant cocoa product (3).

2 g of each product was gently placed on the surface of 50 ml of water at 20 °C in a 50-ml beaker and the time required for substantially sedimenting the whole of the instant cocoa product in water was measured. Each sample was examined four times and the mean was determined. As a result, each composition of the present invention showed excellent dispersion.

EP 0 375 785 B1

| instant cocoa product (1): | 1 min 56 sec, |
| instant cocoa product (2): | 2 min 7 sec, and |
| instant cocoa product (3): | 7 min 2 sec. |

**Claims**

1. A lipid composition having high safety and intense surface activities essentially comprising:
   (A) acylglycerophospholipid(s),
   (B) fatty acid monoglyceride(s),
   (C) fatty acid(s) and
   (D) bile acid(s) and/or alkali salt(s) thereof
   wherein said component (A) comprises acylglycerophospholipid(s) (A-1) containing at least 10% by weight of monoacylglycerophospholipid(s),
   said component (B) comprises fatty acid monoglyceride(s) (B-1) comprising monoglyceride(s) of unsaturated fatty acid(s) having 14 to 22 carbon atoms or mixed fatty acid(s) comprising said unsaturated fatty acid(s) and saturated fatty acid(s) having 14 to 22 carbon atoms,
   and having an iodine value of 30 or above and containing at least 40% by weight of monoester(s),
   said component (C) comprises at least one fatty acid (C-1) selected from the group consisting of fatty acids (C-1-1) comprising unsaturated fatty acid(s) having 16 to 22 carbon atoms and a mixture of said unsaturated fatty acid(s) with saturated fatty acid(s) having 14 to 18 carbon atoms and having an iodine value of 50 or above and medium-chain fatty acid(s) (C-1-2) having 10 to 13 carbon atoms,
   the ratio by weight of these components satisfies the following relationships:

   (B-1)/(A-1) = 10/90 to 90/10,
   (C-1)/(B-1) = 20/100 to 250/100, and
   (C-1)/[(A-1) + (B-1)] = 10/100 to 200/100,

   and when the ratio [(B-1) + (C-1)]/(A-1) exceeds 4, said component (D) is added in an amount of 5 to 100% by weight based on the total amount of (A-1) + (B-1) + (C-1).

2. A lipid composition having high safety and intense surface activities essentially comprising:
   (A) acylglycerophospholipid(s),
   (B) fatty acid monoglyceride(s),
   (C) fatty acid(s) and
   (D) bile acid(s) and/or alkali salt(s) thereof,
   wherein said component (A) comprises lysophosphatidylcholine (A-2),
   said component (B) comprises monoglyceride(s) (B-2) of unsaturated fatty acid(s) having 16 to 22 carbon atoms,
   said component (C) comprises fatty acid(s) (C-2) having 9 to 13 carbon atoms,
   the ratio by weight of these components satisfies the following relationships:

   (B-2)/(A-2) = 10/90 to 90/10,
   (C-2)/(B-2) = 20/100 to 200/100, and
   (C-2)/[(A-2) + (B-2)] = 10/100 to 200/100,

   and when the ratio [(B-2) + (C-2)]/(A-2) exceeds 5, said component (D) is added in an amount of 5 to 100% by weight based on the total amount of (A-2) + (B-2) + (C-2).

3. A lipid composition having high safety and intense surface activities essentially comprising:
   (A) acylglycerophospholipid(s),
   (B) fatty acid monoglyceride(s),
   (C) fatty acid(s) and
   (D) bile acid(s) and/or alkali salt(s) thereof,
   wherein said component (A) comprises acylglycerophospholipid(s) (A-3) containing at least 10% by weight of monoacylglycerophospholipid(s),
   said component (B) comprises monoglyceride(s) (B-3) of fatty acid(s) having 8 to 13 carbon atoms,

58

EP 0 375 785 B1

said component (C) comprises saturated fatty acid(s) having 8 to 18 carbon atoms and/or unsaturated fatty acid(s) having 14 to 22 carbon atoms (C-3),
the ratio by weight of these components satisfies the following relationships:

(B-3)/(A-3) = 10/90 to 95/5,
(C-3)/(B-3) = 20/100 to 250/100, and
(C-3)/[(A-3) + (B-3)] = 10/100 to 200/100,

and when the ratio [(B-3) + (C-3)]/(A-3) exceeds 6, said component (D) is added in an amount of 5 to 100% by weight based on the total amount of (A-3) + (B-3) + (C-3).

**Patentansprüche**

1.  Lipidzusammensetzung mit hoher Sicherheit und starken oberflächenaktivitäten, umfassend im wesentlichen:
    (A) Acylglycerophospholipid(e),
    (B) Fettsäuremonoglycerid(e),
    (C) Fettsäure(n) und
    (D) Gallensäure(n) und/oder Alkalisalz(e) davon,
    wobei der Bestandteil (A) Acylglycerophospholipid(e) (A-1), enthaltend mindestens 10 Gew.-% Monoacylglycerophospholipid(e), umfaßt,
    der Bestandteil (B) Fettsäuremonoglycerid(e) (B-1), umfassend Monoglycerid(e) ungesättigter Fettsäure(n) mit 14 bis 22 Kohlenstoffatomen oder gemischte Fettsäure(n), umfassend diese ungsättigte(n) Fettsäure(n) und gesättigte Fettsäure(n) mit 14 bis 22 Kohlenstoffatomen, mit einer Jodzahl von 30 oder mehr und mindestens 40 Gew.-% Monoester, umfaßt,
    der Bestandteil (C) mindestens eine Fettsäure (C-1), ausgewählt aus der Gruppe, bestehend aus Fettsäuren (C-1-1), umfassend ungesättigte Fettsäure(n) mit 16 bis 22 Kohlenstoffatomen und ein Gemisch aus der (den) Fettsäure(n) mit gesättigter(n) Fettsäure(n) mit 14 bis 18 Kohlenstoffatomen und einer Jodzahl von 50 oder mehr und Fettsäure(n) mittlerer Kettenlänge (C-1-2) mit 10 bis 13 Kohlenstoffatomen umfaßt,
    wobei das Gewichtsverhältnis dieser Bestandteile die folgenden Beziehungen erfüllt:

    (B-1)/(A-1) = 10/90 bis 90/10,
    (C-1)/(B-1) = 20/100 bis 250/100 und
    (C-1)/[(A-1) + (B-1)] = 10/100 bis 200/100

    und wenn das Verhältnis [(B-1) + (C-1)]/(A-1) 4 übersteigt, wird der Bestandteil (D) in einer Menge von 5 bis 100 Gew.-%, bezogen auf die gesamte Menge von [(A-1) + (B-1) + (C-1)], zugefügt.

2.  Lipidzusammensetzung mit hoher Sicherheit und starken Oberflächenaktivitäten, im wesentlichen umfassend:
    (A) Acylglycerophospholipid(e),
    (B) Fettsäuremonoglycerid(e),
    (C) Fettsäure(n) und
    (D) Gallensäure(n) und/oder Alkalisalz(e) davon,
    wobei der Bestandteil (A) Lysophosphatidylcholin (A-2) umfaßt,
    der Bestandteil (B) Monoglycerid(e) (B-2) ungesättigter Fettsäure(n) mit 16 bis 22 Kohlenstoffatomen umfaßt,
    der Bestandteil (C) Fettsäure(n) (C-2) mit 9 bis 13 Kohlenstoffatomen umfaßt,
    das Gewichtsverhältnis dieser Bestandteile die folgenden Beziehungen erfüllt:

    (B-2)/(A-2) = 10/90 bis 90/100
    (C-2)/(B-2) = 20/100 bis 200/100 und
    (C-2)/[(A-2) + (B-2)] = 10/100 bis 200/100,

    und wenn das Verhältnis [(B-2) + (C-2)]/(A-2) 5 übersteigt, wird der Bestandteil (D) in einer Menge von 5 bis 100 Gew.-%, bezogen auf die Gesamtmenge von (A-2) + (B-2) + (C-2), zugefügt.

59

**3.** Lipidzusammensetzung mit hoher Sicherheit und starken Oberflächenaktivitäten, im wesentlichen umfassend:

(A) Acylglycerophospholipid(e),

(B) Fettsäuremonoglycerid(e),

(C) Fettsäure(n) und

(D) Gallensäure(n) und/oder Alkalisalz(e) davon,

wobei der Bestandteil (A) Acylglycerophospholipid(e)

(A-3), enthaltend mindestens 10 Gew.-% Monoacylglycerophospholipid(e), umfaßt,

der Bestandteil (B) Monoglycerid(e) (B-3) von Fettsäure(n) mit 8 bis 13 Kohlenstoffatomen umfaßt,

der Bestandteil (C) gesättigte Fettsäure(n) mit 8 bis 18 Kohlenstoffatomen und/oder ungesättigte Fettsäure(n) mit 14 bis 22 Kohlenstoffatomen (C-3) umfaßt,

das Gewichtsverhältnis dieser Bestandteile die folgenden Beziehungen erfüllt:

(B-3)/(A-3) = 10/90 bis 95/5,

(C-3)/(B-3) = 20/100 bis 250/100 und

(C-3)/[(A-3) + (B-3)] = 10/100 bis 200/100

und wenn das Verhältnis [(B-3) + (C-3)]/(A-3) 6 übersteigt, wird der Bestandteil (D) in einer Menge von 5 bis 100 Gew.-%, bezogen auf die Gesamtmenge von (A-3) + (B-3) + (C-3), zugefügt.

## Revendications

**1.** Composition lipidique ayant une sécurité élevée et des activités de surface intenses, comprenant essentiellement :

(A) un/des acylglycérophospholipide(s),

(B) un/des monoglycéride(s) d'acide(s) gras,

(C) un/des acide(s) gras et

(D) un/des acide(s) biliaire(s) et/ou un/des sel(s) alcalin(s) de celui-ci/ceux-ci

dans laquelle ledit composant (A) comprend un/des acylglycérophospholipide(s) (A-1) contenant au moins 10 % en poids de monoacylglycérophospholipide(s),

ledit composant (B) comprend un/des monoglycéride(s) d'acide(s) gras (B-1) comprenant un/des monoglycéride(s) d'acide(s) gras insaturé(s) ayant 14 à 22 atomes de carbone ou d'acide(s) gras mélangé(s) comprenant ce (ces) acides gras insaturé(s) et un/des acide(s) gras saturé(s) ayant 14 à 22 atomes de carbone, et ayant un indice d'iode de 30 ou plus, et contenant au moins 40 % en poids de monoester(s),

ledit composant (C) comprend au moins un acide gras (C-1) choisi dans le groupe constitué des acides gras (C-1-1) comprenant un/des acide(s) gras insaturé(s) ayant 16 à 22 atomes de carbone, et un mélange dudit/desdits acide(s) gras insaturé(s) avec un/des acide(s) gras saturé(s) ayant 14 à 18 atomes de carbone et ayant un indice d'iode de 50 ou plus, et un/des acide(s) gras à chaîne moyenne (C-1-2) ayant 10 à 13 atomes de carbone,

le rapport en poids de ces composants satisfait les relations suivantes :

(B-1)/(A-1) = 10/90 à 90/10,

(C-1)/(B-1) = 20/100 à 250/100, et

(C-1)/[(A-1) + (B-1)] = 10/100 à 200/100,

et lorsque le rapport [(B-1) + (C-1)]/(A-1) dépasse 4, ledit composent (D) est ajouté en une quantité de 5 à 100 % en poids sur la base de la quantité totale de (A-1) + (B-1) + (C-1).

**2.** Composition lipidique ayant une sécurité élevée et des activités de surface intenses, comprenant essentiellement :

(A) un/des acylglycérophospholipide(s),

(B) un/des monoglycéride(s) d'acide(s) gras,

(C) un/des acide(s) gras et

(D) un/des acide(s) biliaire(s) et/ou un/des sel(s) alcalin(s) de celui-ci/ceux-ci

dans laquelle ledit composant (A) comprend de la lysophosphatidylcholine (A-2),

ledit composant (B) comprend un/des monoglycéride(s) (B-2) d'acide(s) gras insaturé(s) ayant 16 à 22 atomes de carbone,

ledit composent (C) comprend un/des acide(s) gras (C-2) ayant 9 à 13 atomes de carbone,
le rapport en poids de ces composants satisfait les relations suivantes :

(B-2)/(A-2) = 10/90 à 90/10,
(C-2)/(B-2) = 20/100 à 200/100, et
(C-2)/[(A-2) + (B-2)] = 10/100 à 200/100,

et lorsque le rapport [(B-2) + (C-2)]/(A-2) dépasse 5, ledit composent (D) est ajouté en une quantité de 5 à 100 % en poids sur la base de la quantité totale de (A-2) + (B-2) + (C-2).

3. Composition lipidique ayant une sécurité élevée et des activités de surface intenses, comprenant essentiellement :
   (A) un/des acylglycérophospholipide(s),
   (B) un/des monoglycéride(s) d'acide(s) gras,
   (C) un/des acide(s) gras et
   (D) un/des acide(s) biliaire(s) et/ou un/des sel(s) alcalin(s) de celui-ci/ceux-ci
dans laquelle ledit composant (A) comprend un/des acylglycérophospholipide(s) (A-3) contenant au moins 10 % en poids de monoacylglycérophospholipide(s),
ledit composant (B) comprend un/des monoglycéride(s) (B-3) d'acide(s) gras ayant 8 à 13 atomes de carbone,
ledit composant (C) comprend un/des acide(s) gras saturé(s) ayant 8 à 18 atomes de carbone et/ou un/des acide(s) gras insaturé(s) ayant 14 à 22 atomes de carbone (C-3),
le rapport en poids de ces composants satisfait les relations suivantes :

(B-3)/(A-3) = 10/90 à 95/5,
(C-3)/(B-3) = 20/100 à 250/100, et
(C-3)/[(A-3) + (B-3)] = 10/100 à 200/100,

et lorsque le rapport [(B-3) + (C-3)]/(A-3) dépasse 6, ledit composant (D) est ajouté en une quantité de 5 à 100 % en poids sur la base de la quantité totale de (A-3) + (B-3) + (C-3).